# EUROPEAN PATENT APPLICATION

(11) **EP 3 656 306 A2**
(43) Date of publication of application: **27.05.2020**
(21) Application number: 19206452.5
(22) Date of filing: 12.07.2016
(51) Int. Cl.: A61B 6/00, H04W 84/20, H04L 29/12, H04W 48/20, H04W 88/10

(54) **RADIATION IMAGING SYSTEM, METHOD OF CONTROLLING RADIATION IMAGING SYSTEM, AND CONTROL APPARATUS**

(30) Priority: 31.07.2015 JP 2015152690
(62) Divisional of application: 16178945.8
(71) Applicant: CANON KABUSHIKI KAISHA, Ohta-ku Tokyo 146-8501 (JP)
(72) Inventor: ISHIOKA, Toshiya, Ohta-ku, Tokyo 146-8501 (JP)
(74) Representative: TBK

(57) **Abstract**

A radiation imaging system includes a radiation imaging apparatus capable of operating as a master unit or a slave unit in wireless communication, and a control apparatus capable of controlling an operation of the radiation imaging apparatus. The control apparatus includes a determination unit configured to determine whether a device operating as the master unit of the wireless communication exists in the radiation imaging system, and a control unit configured to control the operation of the radiation imaging apparatus based on the determination. The control unit controls the operation of the radiation imaging apparatus based on the determination such that one master unit of the wireless communication exists in the radiation imaging system.

## Description

### BACKGROUND OF THE INVENTION

### Field of the Invention

The present invention relates to a radiation imaging system, a method of controlling the radiation imaging system, and a control apparatus.

### Description of the Related Art

Conventionally, a radiation imaging apparatus that irradiates an object with radiation generated by a radiation source, detects the intensity distribution of the radiation transmitted through the object, and converts it into image data or a radiation imaging system including the radiation imaging apparatus has become widespread.

As methods of obtaining image data by the radiation imaging apparatus, a method using a dedicated film and a method of converting radiation into visible light by a phosphor, converting the visible light into an electrical signal by a photosensor, and outputting digital data are known.

Japanese Patent Laid-Open No. 2013-236711 discloses a radiation imaging system including a radiation imaging apparatus with a wireless function, a master unit for wireless communication (access point (AP)), and a console. The console can be formed as an information processing apparatus (PC) including an output system that gives an operation instruction to the radiation imaging apparatus and displays image data as an imaging result. The access point (AP) is a device that mediates wireless data communication, and can mediate communication between the console and the radiation imaging apparatus.

When the access point (AP) is arranged, the radiation imaging system can incorporate a plurality of wireless devices, and a versatile system can be constructed. On the other hand, as in Japanese Patent Laid-Open No. 2013-236711, the access point (AP) is often provided in the radiation imaging system as a single unit, resulting in an increase in the number of system components. As a measure to cope with this, the radiation imaging apparatus has a function of operating as an access point (AP), thereby reducing the system components.

However, when executing this method, no examination is made at all concerning the assignment of wireless identification information (for example, SSID) for wireless communication to devices that operate as access points (APs). For this reason, the same wireless communication ID may be assigned to a plurality of radiation imaging apparatuses in the radiation imaging system, and a plurality of access points (APs) having the same wireless communication ID may exist in the system. In this case, communication between a console (control apparatus) and a radiation imaging apparatus which should be used in combination may be impossible or unstable.

The present invention provides a radiation imaging technique capable of performing stable communication between a radiation imaging apparatus and a control apparatus.

### SUMMARY OF THE INVENTION

The present invention in its first aspect provides a radiation imaging system as specified in claims 1 to 13.

The present invention in its second aspect provides a method of controlling a radiation imaging system as specified in claim 14.

The present invention in its third aspect provides a control apparatus as specified in claim 15.

According to the present invention, stable communication can be performed between a radiation imaging apparatus and a control apparatus.

Further features of the present invention will become apparent from the following description of exemplary embodiments (with reference to the attached drawings).

### BRIEF DESCRIPTION OF THE DRAWINGS

Fig. 1A is a view showing an example of the arrangement of a radiation imaging system according to the first embodiment;
Fig. 1B is a view showing an example of the arrangement of the radiation imaging system according to the first embodiment;
Fig. 2 is a block diagram showing an example of the arrangement of a radiation imaging apparatus;
Fig. 3 is a flowchart for explaining communication preparation processing of the radiation imaging system;
Figs. 4A and 4B are flowcharts for showing an example of the processing of a console and the radiation imaging apparatus at the time of communication preparation;
Fig. 5 is a flowchart for explaining communication preparation processing when using a plurality of radiation imaging apparatuses;
Figs. 6A and 6B are flowcharts for explaining processing of the console of a radiation imaging system;
Figs. 7A and 7B are flowcharts for explaining processing of the radiation imaging apparatus of the radiation imaging system;
Fig. 8 is a flowchart for explaining communication preparation processing of a radiation imaging system;
Fig. 9 is a flowchart for explaining processing of adding a radiation imaging apparatus to the system;
Fig. 10 is a flowchart for explaining stop processing of a radiation imaging apparatus that is operating in the AP mode; and
Figs. 11A and 11B are flowcharts for showing processing of the console of a radiation imaging system.

### DESCRIPTION OF THE EMBODIMENTS

Embodiments of the present invention will now be described in detail with reference to the accompanying drawings. Note that the constituent elements described in the embodiments are merely examples. The technical scope of the present invention is determined by the scope of claims and is not limited by the following individual embodiments.

As a radiation imaging system, various arrangement examples can be assumed. In the following embodiments, basic arrangement examples will be described, including the combination of the constituent units of a radiation imaging system, timing of assigning, to a radiation imaging apparatus, wireless identification information (wireless ID) necessary to operate as the master unit of wireless communication, and the number of radiation imaging apparatuses that operate as access points (APs). Even for an arrangement example whose detailed description will be omitted, contents described in the embodiments can be applied.

### (First Embodiment)

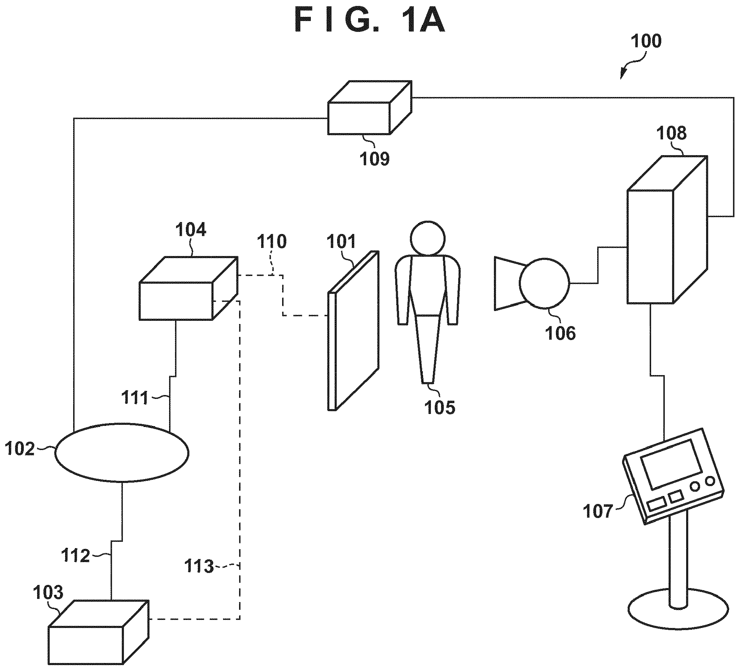

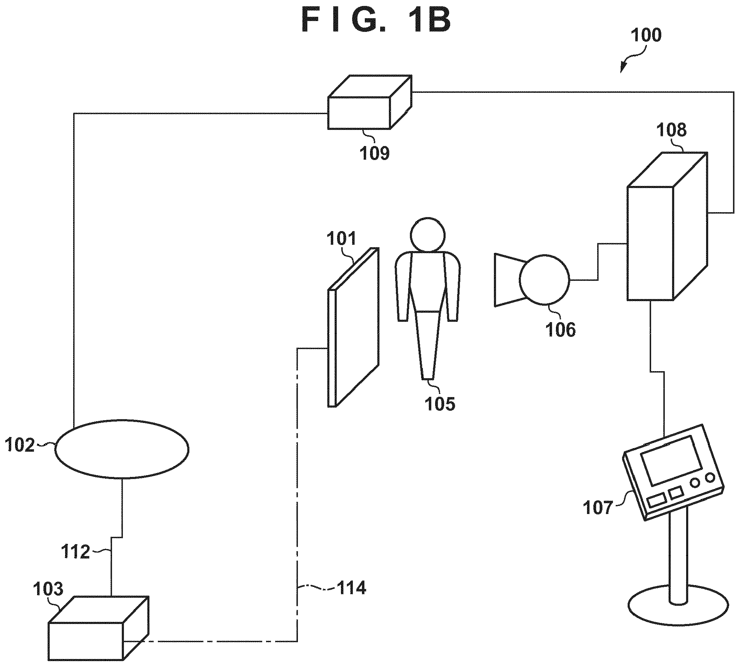

In this embodiment, a system arrangement in which one console and one radiation imaging apparatus exist will be described as an example of the arrangement of a radiation imaging system. The radiation imaging system includes a radiation imaging apparatus capable of operating as a master unit or slave unit of wireless communication, and a control apparatus capable of controlling the operation of the radiation imaging apparatus. In the radiation imaging system, the radiation imaging apparatus can operate as an access point (AP: master unit) in wireless communication. The radiation imaging apparatus can also operate as a slave unit in wireless communication. Fig. 1A shows an example of the arrangement of the radiation imaging system according to the first embodiment in which the radiation imaging apparatus operates as a slave unit in wireless communication. Fig. 1B shows an example of the arrangement of the radiation imaging system according to the first embodiment in which the radiation imaging apparatus operates as a master unit in wireless communication. The arrangement of a radiation imaging system 100 will be described below with reference to Figs. 1A and 1B.

Referring to Fig. 1A, a radiation imaging apparatus 101 has a wireless communication function, and can exchange various kinds of information including image data with a console 103 via communication channels 110 to 113. As an example, the radiation imaging apparatus 101 can transmit, for example, a radio field intensity from the access point, the temperature information of the imaging apparatus, the remaining life information of a battery provided in the radiation imaging apparatus 101, and the like to the console 103 as well as image data. The console 103 is constructed by, for example, a control apparatus (PC) having a display function such as a display unit and an input function from a user via an input unit. The console 103 can transmit an instruction from the user to the radiation imaging apparatus 101, or receive image data acquired by the radiation imaging apparatus 101 and present it to the user. The console 103 (control apparatus) may have a wired communication function in addition to the wireless communication function.

### (Communication between Radiation Imaging Apparatus 101 and Console 103)

Upon acquiring image data, the radiation imaging apparatus 101 transmits the image data to the console 103 via one of the communication channels 110 to 113 according to the arrangement state of the system. Arrangements and communication channels at the time of image data acquisition of the radiation imaging apparatus 101 will be described here in correspondence with each condition.

As an example, a case in which the radiation imaging apparatus 101 operating as a slave unit is communicable with the console 103 via an AP unit 104 operating as a master unit in wireless communication will be described.

At this time, the radiation imaging apparatus 101 transmits image data to the AP unit 104 formed in the system using the wireless communication function of its own. The AP unit 104 transmits the received image data to the console 103 via a network 102. At this time, the image data is transmitted from the radiation imaging apparatus 101 to the console 103 via the communication channel 110, the AP unit 104, the communication channel 111, the network 102, and the communication channel 112. The communication channels 111 and 112 and the network 102 which exist between the AP unit 104 and the console 103 can be configured by either wired communication or wireless communication. That is, out of the communication between the radiation imaging apparatus 101 and the console 103, at least information exchange between the radiation imaging apparatus 101 and the AP unit 104 is performed by wireless communication. The communication channels 111 and 112 and the network 102 can also be configured using both wired communication and wireless communication.

The AP unit 104 can also be connected to the console 103 directly without an intervention of the network 102. At this time, the channel to transmit image data from the radiation imaging apparatus 101 to the console 103 is formed from the communication channel 110, the AP unit 104, and the communication channel 113. The communication channel 113 can be configured by either wired communication or wireless communication, like the network 102.

A case in which the radiation imaging apparatus 101 operates as an access point (AP: master unit) will be described next with reference to Fig. 1B. The same reference numerals as in Fig. 1A denote constituent elements of the same functions in Fig. 1B. The radiation imaging apparatus 101 has a wireless communication function, and can execute image data exchange directly with the console 103 (communication channel 114). The console 103 is constructed by, for example, a control apparatus (PC) having a display function such as a display unit and an input function from a user via an input unit. The console 103 can transmit an instruction from the user to the radiation imaging apparatus 101 by direct wireless communication, or receive image data acquired by the radiation imaging apparatus 101 by direct wireless communication from the radiation imaging apparatus 101, and present it to the user.

The radiation imaging apparatus 101 can perform direct wireless communication with the console 103 using the wireless communication function, and directly transmit image data to the console 103. The wireless communication channel at this time is the communication channel 114. Note that Fig. 1B shows an example in which image data is transmitted from the radiation imaging apparatus 101 to the console 103 by direct wireless communication. However, networks using wireless communication or using both wireless communication and wired communication can also intervene between the radiation imaging apparatus 101 and the console 103 to transmit the image data.

Communication channels used to execute image data exchange between the radiation imaging apparatus 101 and the console 103 have been described above. Note that in the arrangement examples shown in Figs. 1A and 1B, an example in which the wireless communication function is used to transmit image data from the radiation imaging apparatus 101 has been described. However, this embodiment is not limited to this example, and for example, the radiation imaging apparatus 101 can also execute communication with another unit by wired communication.

### (Radiation Imaging)

A procedure of imaging of an object 105 by the radiation imaging apparatus 101 will be described next. Before executing imaging of the object 105, the radiation imaging apparatus 101 is set at a position where it is irradiated with radiation emitted by a radiation tube 106 and transmitted through the object 105. As for the procedure of imaging, after the user activates the radiation imaging apparatus 101, the radiographer operates the console 103 to set the radiation imaging apparatus 101 in an imaging enable state. Next, the radiographer operates a console 107 of a radiation generation apparatus 108 to set radiation irradiation conditions. After the end of the above-described processing, the radiographer confirms that imaging preparations including the object 105 are completed, and presses an exposure switch provided on the console 107 of the radiation generation apparatus 108 to do radiation exposure.

At the time of radiation exposure, the radiation generation apparatus 108 notifies, via a connector 109 (radiation device connector) or a network, the radiation imaging apparatus 101 of a signal representing that radiation irradiation starts. In the arrangement examples shown in Figs. 1A and 1B, the radiation generation apparatus 108 and the radiation imaging apparatus 101 are connected via the connector 109 and the network 102. However, the connection form is not limited to this example. The irradiation notification may be unnecessary depending on the function of the radiation imaging apparatus 101.

Upon receiving the signal indicating radiation irradiation, the radiation imaging apparatus 101 confirms whether it is ready for radiation irradiation. If there is no problem, the radiation imaging apparatus 101 transmits an irradiation permission signal to the radiation generation apparatus 108. After receiving the irradiation permission signal from the radiation imaging apparatus 101, the radiation generation apparatus 108 emits radiation.

Upon receiving a radiation irradiation completion signal from the radiation generation apparatus 108, the radiation imaging apparatus 101 starts generating image data and transmits the generated image data to the console 103 via the above-described communication channel. Upon receiving the image data from the radiation imaging apparatus 101, the console 103 displays the received image data on the display unit of the console 103.

### (Internal Arrangement of Radiation Imaging Apparatus 101)

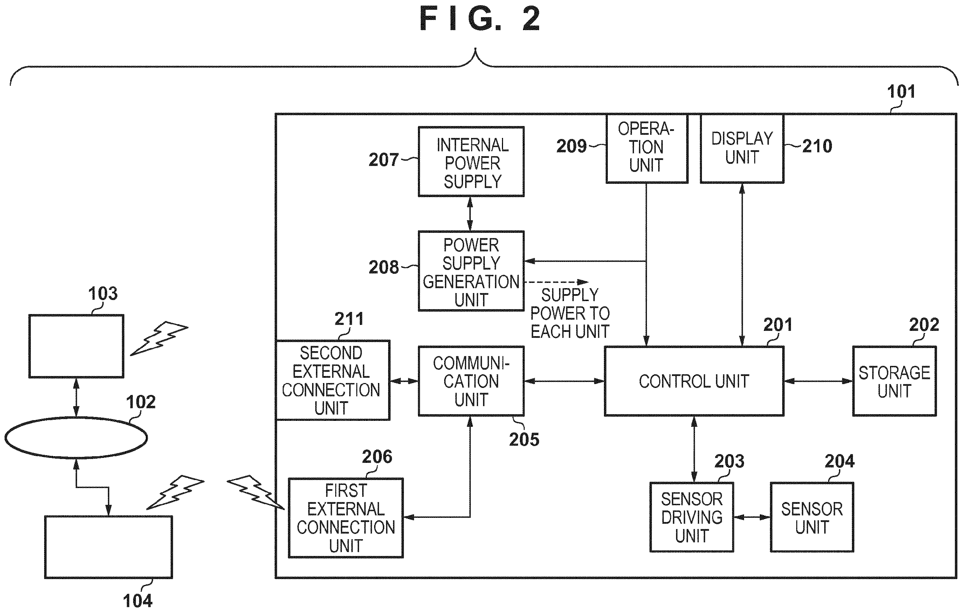

An example of the internal arrangement of the radiation imaging apparatus 101 will be described next with reference to Fig. 2. The radiation imaging apparatus 101 includes a sensor unit 204 configured to change incident radiation into an electrical signal. The sensor unit 204 is formed by a scintillator and a photodetector array. The scintillator and the photodetector array have two-dimensional planar shapes and are arranged with the planes facing each other. The scintillator is excited by radiation such as X-rays and generates visible light. Each photodetector converts the visible light into an electrical signal.

A sensor driving unit 203 drives the sensor unit 204 with the above-described arrangement. The sensor driving unit 203 selects a row or column from which an electrical signal is to be extracted, amplifies an extracted electrical signal, or supplies power to the photodetector array. The sensor driving unit 203 transmits the electrical signal extracted from the photodetector array to a control unit 201. Upon receiving the electrical signal from the sensor driving unit 203, the control unit 201 outputs the received electrical signal to a storage unit 202 and saves the signal in the storage unit 202. As for the arrangement of the sensor unit 204, for example, the type of the scintillator and the type of the photodetector are not particularly limited, and various arrangements can be used.

The control unit 201 performs processing associated with control of each unit of the radiation imaging apparatus 101. For example, concerning imaging, the control unit 201 can output an instruction to drive the sensor unit 204 to the sensor driving unit 203, save obtained image data in the storage unit 202, or extract image data from the storage unit 202. The control unit 201 can also transmit image data to another device via a communication unit 205, receive an instruction from another device via the communication unit 205, or control activation/stop of the radiation imaging apparatus 101 based on an operation from an operation unit 209. The control unit 201 can also notify the user of the operation state or error state of the radiation imaging apparatus 101 via a display unit 210. In this embodiment, the above-described processing contents are processed by one control unit 201. However, the processing can also divisionally be performed by a plurality of control units, for example, two or more control units.

The storage unit 202 can save image data acquired by the radiation imaging apparatus 101 or log information representing an internal processing result, or the like. If the control unit 201 uses software, the storage unit 202 can also store the software used by the control unit 201. As the arrangement of the storage unit 202, for example, volatile/nonvolatile memories, HDDs, and the like can be mounted in the radiation imaging apparatus 101 in various combinations. In this embodiment, only one storage unit 202 is illustrated in Fig. 2. However, a plurality of storage units can also be arranged.

The communication unit 205 performs processing for implementing communication between the radiation imaging apparatus 101 and another device. The communication unit 205 according to this embodiment is connected to a first external connection unit 206 for wireless communication, and can communicate with the AP unit 104 or the console 103 via the first external connection unit 206. An example of the first external connection unit 206 is an antenna for wireless communication. Note that the communication is not limited to this form, as described above, and an arrangement with a wired communication function may be employed. The standard and method of the communication are not particularly limited.

The communication unit 205 is also connected to a second external connection unit 211. The second external connection unit 211 is used to exchange various kinds of information with another device such as a console independently of communication via the first external connection unit 206. An example of the information is setting information of the radiation imaging apparatus 101 when communicating via the first external connection unit 206. The connection method of the second external connection unit 211 can be either wired communication or wireless communication. In wired communication, a cable may be connected or a storage medium is directly assembled in the radiation imaging apparatus 101. In wireless communication as well, the method is not particularly limited, and communication using light or a radio wave is usable. In the arrangement example shown in Fig. 2, the communication unit 205 is connected to two external connection units (the first external connection unit 206 and the second external connection unit 211). However, a dedicated communication unit may be provided in correspondence with each connection unit.

When communicating with the console 103 or the AP unit 104 via the first external connection unit 206, the radiation imaging apparatus 101 can select an operation mode of communication in the radiation imaging apparatus 101, that is, whether to operate as an access point (AP: master unit) or a slave unit. The radiation imaging apparatus 101 can execute communication using wireless identification information (wireless ID) according to the selected operation mode. A method of setting the wireless identification information (wireless ID) will be described later. Note that the operation mode need not always be selected by the radiation imaging apparatus 101, and the console 103 (control apparatus) can also control selection of the operation mode.

Operation modes available in wireless communication of the radiation imaging apparatus according to each embodiment include at least a master unit mode (access point mode: to be referred to as an AP mode hereinafter) and a slave unit mode (station mode: to be referred to as an STA mode hereinafter). The AP mode is a mode in which the radiation imaging apparatus directly wirelessly communicates with the control apparatus and also relays wireless communication between the control apparatus and another radiation imaging apparatus. The STA mode is a mode in which the radiation imaging apparatus does not directly communicate with the control apparatus and performs wireless communication with the control apparatus via other devices including another radiation imaging apparatus.

The radiation imaging apparatus 101 includes an internal power supply 207. The internal power supply 207 can be formed as, for example, a detachable rechargeable battery. However, this embodiment is not limited to this example, and various power supplies such as a rechargeable power supply, an unrechargeable power supply, a detachable power supply, an undetachable power supply, and a power generation method can be used in combination. A power supply generation unit 208 generates a voltage/current needed by each unit of the radiation imaging apparatus 101 from power given by the internal power supply 207, and distributes the voltage/current.

The operation unit 209 accepts an operation input from the user. The operation unit 209 can be formed by, for example, various kinds of switches, a touch panel, and the like to be operated by the user. A receiving unit that accepts an input from a remote controller dedicated to operations can be arranged together.

The display unit 210 is used to notify the user of the state of the radiation imaging apparatus 101 or the like. The display unit 210 can be formed by, for example, an LED, an LCD, a monitor, and the like. Note that as a notification method for the user, a notification unit such as a speaker can be arranged together.

### (Communication Establishment Operation)

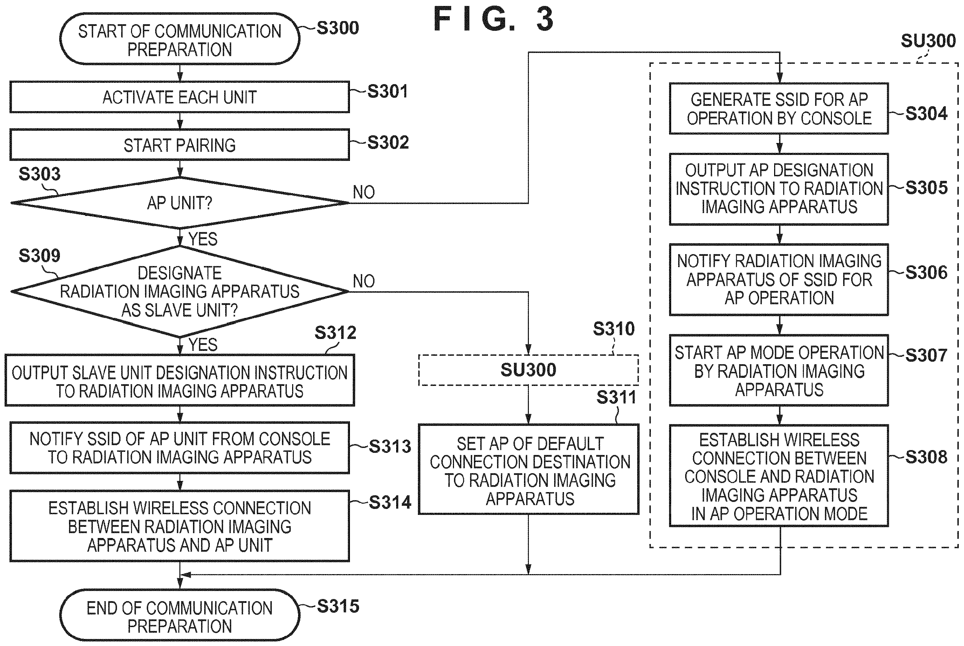

A communication establishment operation in a radiation imaging environment will be described next with reference to Figs. 2 and 3. Fig. 3 is a flowchart showing communication preparation processing when incorporating the radiation imaging apparatus 101 in the radiation imaging system 100. A description will be made here assuming that wireless communication is a wireless LAN.

A case in which a radiation image is captured by the radiation imaging system 100 including the radiation imaging apparatus 101, the console 103, and the AP unit 104 described with reference to Fig. 2 will be explained. The radiation imaging apparatus 101 is assumed to have a function of performing imaging upon automatically detecting radiation irradiation. The connector 109 described with reference to Fig. 1A is omitted in the arrangement for capturing a radiation image.

In step S300, communication preparation processing starts. When starting imaging, activation of each unit and pairing between the console 103 and the radiation imaging apparatus 101 are started, as indicated by steps S301 and S302 of Fig. 3. Pairing is processing for linking the radiation imaging apparatus 101 and the console 103.

In the arrangement including the AP unit 104 existing in the wireless system, a plurality of wireless communication units (to be also simply referred to as units hereinafter) included in the radiation imaging system can be connected to one AP unit 104. Under the imaging environment, a plurality of consoles and radiation imaging apparatuses each having a wireless communication function may exist. If a console and a radiation imaging apparatus are not notified of the partner units of the pair by pairing, image data acquired by the radiation imaging apparatus may be transmitted to an undesirable console, or it may be impossible to provide the image data to the radiographer as the user because of the unknown transmission destination.

As examples of the pairing method, information exchange by infrared communication, information exchange by NFC (Near Field Communication), and information exchange by a wireless communication function such as Bluetooth® other than the wireless LAN are possible. Assume that communication functions for various kinds of communication are provided on the side of the console 103, and the second external connection unit 211 in the radiation imaging apparatus 101 is formed as a transmitting/receiving unit such as an antenna for various kinds of communication. The radiation imaging apparatus 101 notifies the console 103 of a pairing request based on an operation input to instruct pairing from the operation unit 209, and the console 103 transmits necessary information in response to the pairing request, thereby executing pairing.

As another example of the arrangement for executing pairing, pairing can be executed by physical connection. For example, the console 103 and the second external connection unit 211 may be connected by a cable and notified of each other's information by connection detection. Pairing is not limited to the above-described methods, and can be implanted by a plurality of methods such as a method of causing the radiographer to input the information of partners using the console 103 and the operation unit 209 of the radiation imaging apparatus 101.

When pairing starts in step S302, the console 103 determines the presence/absence of the AP unit 104 in step S303. The console 103 (control apparatus) determines whether a device operating as the master unit of wireless communication exists in the radiation imaging system, and controls the operation of the radiation imaging apparatus based on the determination. The console 103 (control apparatus) can control the operation of the radiation imaging apparatus based on the determination such that one master unit of wireless communication is obtained in the radiation imaging system.

More specifically, based on the determination, if a device (for example, the AP unit 104) operating as the master unit does not exist, the console 103 (control apparatus) makes the radiation imaging apparatus operate as the master unit of wireless communication. If a device operating as the master unit exists, the console 103 (control apparatus) makes the radiation imaging apparatus operate as a slave unit of wireless communication. The console 103 (control apparatus) can control the operation mode of communication in the radiation imaging apparatus based on the presence/absence of the master unit of wireless communication in the radiation imaging system. That is, the console 103 can select, based on the determination result, whether to make the paired radiation imaging apparatus 101 as an access point (AP: master unit) (AP mode) or a slave unit (STA mode). That is, the console 103 (control apparatus) can change the instruction to be given to the radiation imaging apparatus 101 based on the result of determining the presence/absence of the AP unit 104. As an example of the method of determining the presence/absence of the AP unit 104, the numerical range of the host portion is determined in advance out of the IP addresses of the AP unit 104. The console 103 can execute the determination by confirming whether a unit operating by the host for the AP unit 104 exists in the same network.

If it is determined in step S303 of Fig. 3 that the AP unit 104 does not exist in the radiation imaging system (NO in step S303), the process advances to step S304.

In step S304, the console 103 generates an SSID as unique wireless identification information (wireless ID) necessary for the radiation imaging apparatus 101 to operate as an AP. The console 103 (control apparatus) generates unique wireless identification information, and controls the operation of the radiation imaging apparatus as the master unit of wireless communication based on the unique wireless identification information. In this system, since a plurality of radiation imaging apparatuses 101 may be incorporated in the system, an SSID is generated as unique wireless identification information (wireless ID) .

The console 103 (control apparatus) can generate the unique wireless identification information based on character information capable of identifying the device operating as the master unit of wireless communication, unique identification information of the control apparatus, and unique identification information of the radiation imaging apparatus. As the unique wireless identification information, the console 103 (control apparatus) can generate, for example, information including information for specifying the radiation imaging apparatus, information for specifying the timing of pairing between the radiation imaging apparatus and the control apparatus, and information for specifying the control apparatus. More specifically, as an example of the structure of the SSID, the console 103 can generate an SSID such that it includes information (character string) for specifying the radiation imaging apparatus operating as an access point (AP) which is a device having an SSID, information (information representing a year/month/day and time) for specifying the timing of pairing between the radiation imaging apparatus and the control apparatus, and the serial number (information for specifying the control apparatus) of the paired console. By generating such a unique SSID, the possibility of crosstalk occurrence or the like can be lowered even in a state in which a plurality of radiation imaging apparatuses operate as APs.

When character information (character string) representing the radiation imaging apparatus operating as an AP is included in the structure of the SSID, which one of the AP unit 104 and the radiation imaging system operating as an AP should be given priority can be determined. In addition, when the year/month/day and time of pairing is included in the structure of the SSID, the console can preferentially be connected to the latest AP. Furthermore, when the serial number of the console is included in the structure of the SSID, it is possible to prevent identical SSIDs from being generated even under an environment where a plurality of pairs of consoles and radiation imaging apparatuses exist. The SSID need not always include all pieces of information as described above, as a matter of course. If the advantage obtained by including the pieces of information is unnecessary, the console 103 can generate the SSID while excluding the pieces of information. A unique numerical value used in a worldwide range, such as a MAC address provided for a device using Ethernet®, can also be used.

When a unique SSID is generated in step S304, the process advances to step S305. In step S305, the console 103 outputs an instruction (AP designation instruction) to the radiation imaging apparatus 101 to make it operate in the AP mode. In step S306, the console 103 notifies the radiation imaging apparatus 101 of the SSID generated in step S304. In step S307, the radiation imaging apparatus 101 that has received the instruction and the SSID information from the console 103 starts an operation (AP mode operation) as an access point (AP) using the received SSID. After that, in step S308, wireless connection between the console 103 and the radiation imaging apparatus in the AP mode is established. When a predetermined time elapses from the SSID notification or the console 103 receives a preparation completion notification from the radiation imaging apparatus 101 that has started the operation in the AP mode, wireless connection (wireless communication) between the console 103 and the radiation imaging apparatus 101 notified of the SSID is established, thus completing the wireless communication preparation.

On the other hand, upon determining by the determination processing of step S303 that the AP unit 104 that is operating exists in the radiation imaging system (YES in step S303), the process advances to step S309.

When the process advances to step S309, the console 103 (control apparatus) determines whether to designate the radiation imaging apparatus as a slave unit. That is, the console 103 (control apparatus) selects, based on an operation input, whether to make the radiation imaging apparatus operate as a slave unit or master unit of wireless communication. More specifically, the console 103 displays, on the monitor, a window to confirm with the user about the manner the paired radiation imaging apparatus 101 is made to operate, and prompts the radiographer as the user to make selection. If making the radiation imaging apparatus 101 operate as an access point (AP: master unit) is selected as the result of selection of the radiographer, the process advances to step S310. When making the radiation imaging apparatus operate as the master unit is selected, the console 103 (control apparatus) notifies the radiation imaging apparatus of unique wireless identification information, and makes the radiation imaging apparatus operate as the master unit of wireless communication.

On the other hand, if making the radiation imaging apparatus 101 operate as a slave unit is selected by the radiographer in the determination of step S309, the process advances to step S312. When making the radiation imaging apparatus operate as a slave unit of wireless communication is selected, the console 103 (control apparatus) notifies the radiation imaging apparatus of the wireless identification information of a device operating as the master unit, and makes the radiation imaging apparatus operate as a slave unit of the device. In this way, the console 103 (control apparatus) can control the operation of the radiation imaging apparatus based on the selection in step S309.

The processing contents of step S310 are the same as those of steps S304 to S308 described above. In Fig. 3, these processes are defined as SU300 that replaces the description in step S310. When the process of SU300 in step S310 ends, two access points (APs) that are operating exist in the system. In step S311, the console 103 sets the access point (AP) as the default connection destination of itself to the radiation imaging apparatus 101, and completes the communication preparation. In this step, the console 103 (control apparatus) changes the default connection destination of the master unit in the radiation imaging system to the radiation imaging apparatus.

On the other hand, when the process advances to step S312, the console 103 notifies the radiation imaging apparatus 101 of an instruction (slave unit designation instruction) to do an operation as a slave unit. In step S313, the console 103 notifies the radiation imaging apparatus 101 operating as an access point (AP) of the SSID of the AP unit 104. In step S314, wireless connection is established between the radiation imaging apparatus 101 and the AP unit 104, thus completing the communication preparation.

Note that in this embodiment, whether to make the paired radiation imaging apparatus 101 operate as an access point (AP: master unit) (AP mode) or a slave unit can be selected. However, this embodiment is not limited to this example. For example, it is possible to do presetting to make the radiation imaging apparatus 101 incorporated in the system later operate in the AP mode as an access point (AP) to be preferentially connected. It is also possible to do presetting to make the radiation imaging apparatus 101 operate as a slave unit (operate in the STA mode) in a case in which the AP unit 104 exists.

### (Example of Processing of Console and Radiation Imaging Apparatus When Wireless Communication Operation Is Unstable)

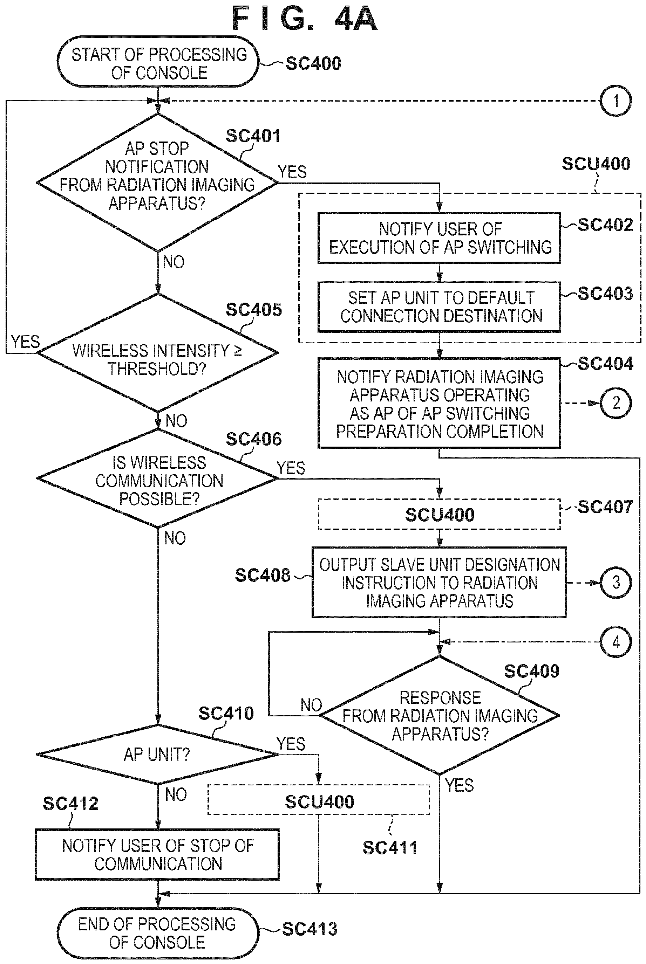

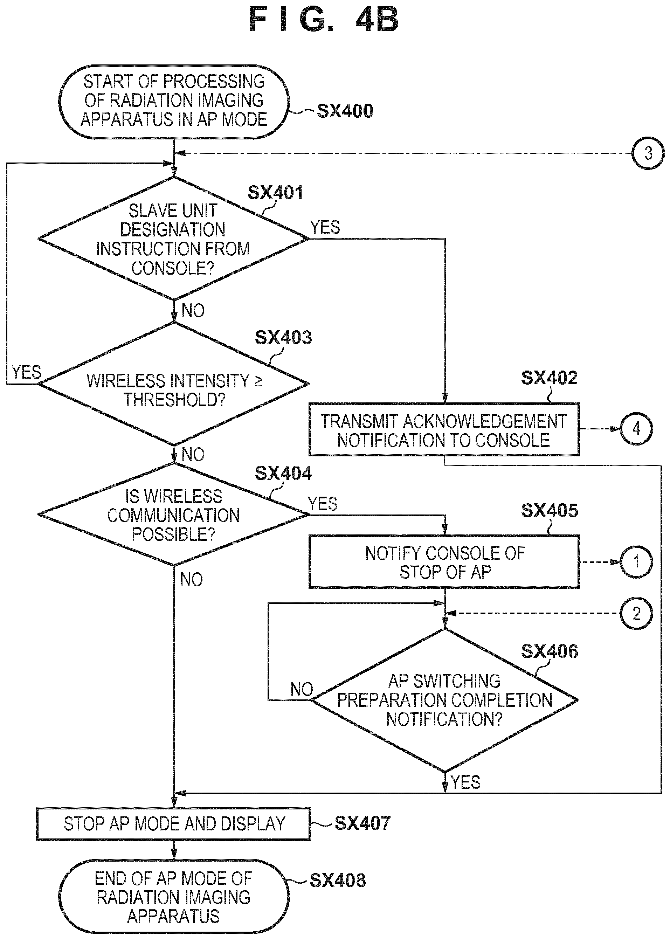

Processing of the radiation imaging apparatus 101 and the console 103 in a case in which the radiation imaging apparatus 101 is carried to a place apart from the system, or the operation becomes unstable when the radiation imaging apparatus 101 is operating as an access point (AP) will be described next with reference to Figs. 4A and 4B.

Figs. 4A and 4B are flowcharts for showing the processing of the console 103 and the radiation imaging apparatus 101 when the radiation imaging apparatus 101 is operating in the AP mode, in which the procedure of processing of the console 103 is shown in Fig. 4A, and the procedure of processing of the radiation imaging apparatus 101 is shown in Fig. 4B. For a portion where they need information exchange, processes are connected by an arrow of a broken line or an alternate long and short dashed line. A step number in the flowchart on the side of the console 103 is indicated by "SC", and a step number in the flowchart on the side of the radiation imaging apparatus 101 is indicated by "SX".

### (Processing of Console 103)

Processing (SC400) of the console 103 will be described first. In step SC401, if the radiation imaging apparatus 101 operating in the AP mode exists in the radiation imaging system, the console 103 monitors the presence/absence of a notification (AP stop notification) to stop the operation as an access point (AP) from the radiation imaging apparatus 101. Note that the condition to transmit the notification (AP stop notification) from the radiation imaging apparatus 101 will be described concerning processing on the side of the radiation imaging apparatus 101 to be described later. Upon receiving the notification (AP stop notification) from the radiation imaging apparatus 101 (YES in step SC401), the console 103 advances the process to step SC402.

In step SC402, the console 103 notifies the radiographer as the user of execution of access point (AP) switching (execution of AP switching) by display on the display unit such as a monitor.

In step SC403, the console 103 sets the AP unit 104 to the default connection destination. In step SC404, the console 103 notifies the radiation imaging apparatus 101 presently operating as an access point (AP) that access point (AP) switching preparation is completed, and the AP mode of the radiation imaging apparatus 101 ends. Based on the end of the AP mode, the operation mode of the radiation imaging apparatus 101 is switched to the mode (STA mode) in which it operates as a slave unit (steps SX406 to SX408). The access point (AP) switching preparation completion notification in step SC404 is transmitted from the console 103 to the radiation imaging apparatus 101. The processing of the radiation imaging apparatus 101 in step SX406 to be described later is executed based on the access point (AP) switching preparation completion notification transmitted from the console 103 to the radiation imaging apparatus 101.

The description has been made here assuming that the AP unit 104 exists in the radiation imaging system when the AP stop notification is transmitted from the radiation imaging apparatus 101. However, this embodiment is not limited to this example. For example, the console 103 can execute processing of determining the presence/absence of the AP unit 104, as in step SC410. That is, according to the presence/absence of the master unit of wireless communication in the radiation imaging system, the console 103 (control apparatus) can control the operation mode of communication in the radiation imaging apparatus based on the processes of steps SC402 to SC404. When the process of step SC404 ends, the process advances to step SC413, and the processing of the console ends. By executing this processing, the console 103 ends the AP mode of the radiation imaging apparatus 101. The AP mode operation of the radiation imaging apparatus 101 operating as an access point (AP) ends.

On the other hand, if the AP stop notification from the radiation imaging apparatus 101 is not received (NO in step SC401), the console 103 advances the process to step SC405. In step SC405, the console 103 confirms the wireless intensity (wireless sensitivity) between the console 103 and the radiation imaging apparatus 101 that is the connection destination operating in the AP mode. If the wireless communication intensity between the control apparatus and the radiation imaging apparatus is lower than a threshold, the console 103 (control apparatus) can make the radiation imaging apparatus operating as the master unit (operating in the AP mode) operate as a slave unit (operate in the STA mode).

Upon determining that the wireless intensity (wireless sensitivity) is equal to or higher than the threshold, and wireless communication can stably be performed (YES in step SC405), the console 103 returns the process to step SC401 to repeat the same processing. On the other hand, if the wireless intensity (wireless sensitivity) is lower than the threshold in step SC405, that is, if the console 103 determines that a stable wireless intensity (wireless sensitivity) cannot be ensured because, for example, the distance between the console 103 and the radiation imaging apparatus 101 increases, a structure that can act as an obstacle suppresses the radio wave, or a device that radiates another electromagnetic wave exerts influence (NO in step SC405), the process advances to step SC406. The threshold used here can be set to a lower limit intensity (lower limit sensitivity) at which wireless communication is possible or a value with a margin to some extent with respect to the lower limit intensity (lower limit sensitivity). Alternatively, instead of using the intensity value (sensitivity value) at the time of determination, the console 103 acquires a change in the intensity value (sensitivity value) from a plurality of intensity values (sensitivity values) that change time-serially, and speculates based on the change in the intensity value (sensitivity value) that the distance between the console 103 and the radiation imaging apparatus 101 is increasing. The console 103 then predicts that the wireless intensity (sensitivity) becomes equal to or lower than the threshold in a predetermined time, and can advance the process to step SC406 after the elapse of the predetermined time.

In step SC406, the console 103 determines whether wireless communication with the radiation imaging apparatus 101 is possible. Even in a case in which the wireless intensity is equal to or lower than the threshold, as described above, if the threshold is a numerical value including a margin, communication may be continued, although it may become unstable.

If the console 103 determines in step SC406 that wireless communication is possible (YES in step SC406), the process advances to step SC407. The processing contents of step SC407 are the same as those of steps SC402 and SC403 described above. In Fig. 4A, these processes are defined as SCU400 that replaces the description in step SC407. When the process of SCU400 in step SC407 ends, the process advances to step SC408. In step SC408, the console 103 notifies the radiation imaging apparatus 101 of an instruction (slave unit designation instruction) to do an operation as a slave unit. The slave unit designation instruction of the console 103 is transmitted from the console 103 to the radiation imaging apparatus 101. The processing of the radiation imaging apparatus 101 in step SX401 to be described later is executed based on the slave unit designation instruction notification transmitted from the console 103 to the radiation imaging apparatus 101.

In step SC409, the console 103 determines whether a response to the slave unit designation instruction notification is received from the radiation imaging apparatus 101. If a response to the slave unit designation instruction notification is not received (NO in step SC409), the console 103 stands by in a state to wait for a response. On the other hand, if a response to the slave unit designation instruction notification is received (YES in step SC409), the process advances to step SC413 to end the processing of the console. By executing this processing, the console 103 ends the AP mode of the radiation imaging apparatus 101. The AP mode operation of the radiation imaging apparatus 101 operating as an access point (AP) ends.

On the other hand, if the console 103 determines in step SC406 that wireless communication is impossible (NO in step SC406), the process advances to step SC410. In step SC410, the console 103 executes processing of determining the presence/absence of the AP unit 104. As for the method of determining the presence/absence of the AP unit 104, the console 103 can determine the presence/absence of the AP unit 104 in accordance with the same procedure as that described concerning the process of step S303 in Fig. 3.

Upon determining in step SC410 that the AP unit 104 that is operating exists in the radiation imaging system (YES in step SC410), the process advances to step SC411.

The processing contents of step SC411 are the same as those of steps SC402 and SC403 described above. In Fig. 4A, these processes are defined as SCU400 that replaces the description in step SC411. When the process of SCU400 in step SC411 ends, the process advances to step SC413. That is, the console 103 notifies the radiographer as the user of execution of access point (AP) switching (execution of AP switching) by display on the display unit such as a monitor (step SC402), and sets the AP unit 104 to the default connection destination (step SC403). In step SC413, the processing of the console ends. By executing this processing, the console 103 ends the AP mode of the radiation imaging apparatus 101. The AP mode operation of the radiation imaging apparatus 101 operating as an access point (AP) ends.

On the other hand, upon determining in step SC410 that the AP unit 104 does not exist in the radiation imaging system (NO in step SC410), the process advances to step SC412. In step SC412, the console 103 notifies the radiographer as the user that a usable access point (AP) does not exist in the radiation imaging system, and then ends the processing.

After execution of the process of step SC412, the processing is ended without considering the possibility of reestablishment of wireless communication with the radiation imaging apparatus 101 operating as an access point (AP). However, the processing of this embodiment is not limited to this example. For example, the console 103 can also execute processing of periodically searching for an access point (AP) in the radiation imaging system at a predetermined time interval in consideration of the possibility of reestablishment.

### (Processing of Radiation Imaging Apparatus 101)

Processing (SX400) of the radiation imaging apparatus 101 operating in the AP mode will be described next. In step SX401, the radiation imaging apparatus 101 determines the presence/absence of a slave unit designation instruction notification from the console 103. The slave unit designation instruction notification is transmitted from the console 103 (step SC408) to the radiation imaging apparatus 101 when the console 103 detects a decrease in the wireless intensity (sensitivity), as described above. If the radiation imaging apparatus 101 determines in step SX401 that the slave unit designation instruction notification is received from the console 103 (YES in step SX401), the process advances to step SX402.

In step SX402, the radiation imaging apparatus 101 transmits an acknowledgement notification to the received slave unit designation instruction notification to the console 103. In step SX407, the radiation imaging apparatus 101 stops the AP mode operation and controls the display on the display unit to notify the radiographer as the user that the AP mode has stopped. In step SX408, the radiation imaging apparatus 101 ends the AP mode.

On the other hand, if the radiation imaging apparatus 101 determines in step SX401 that the slave unit designation instruction notification is not received from the console 103 (NO in step SX401), the process advances to step SX403.

In step SX403, the radiation imaging apparatus 101 operating in the AP mode confirms the wireless intensity (wireless sensitivity) between the radiation imaging apparatus 101 and the console 103 that is the connection destination. This process is the same as that of the console 103 in step SC405. Upon determining that the wireless intensity (wireless sensitivity) is equal to or higher than the threshold, and wireless communication can stably be performed (YES in step SX403), the radiation imaging apparatus 101 returns the process to step SX401 to repeat the same processing.

On the other hand, if the wireless intensity (wireless sensitivity) is lower than the threshold in step SX403, the process advances to step SX404. In step SX404, the radiation imaging apparatus 101 determines whether wireless communication with the console 103 is possible. As described concerning step SC406 of the console 103, even in a case in which the wireless intensity is equal to or lower than the threshold, if the threshold is a numerical value including a margin, communication may be continued, although it may become unstable.

If the radiation imaging apparatus 101 determines in step SX404 that wireless communication is possible (YES in step SX404), the process advances to step SX405.

In step SX405, the radiation imaging apparatus 101 transmits a notification (AP stop notification) to stop the operation as an access point (AP) to the console 103. The AP stop notification is transmitted from the radiation imaging apparatus 101 to the console 103. The processing of the console 103 in step SC401 is executed based on the AP stop notification transmitted from the radiation imaging apparatus 101 to the console 103.

In step SX406, the radiation imaging apparatus 101 determines the presence/absence of an access point (AP) switching preparation completion notification transmitted from the console 103 as a reply to the transmitted AP stop notification.

Upon determining in step SX406 that the access point (AP) switching preparation completion notification is not received (NO in step SX406), the radiation imaging apparatus 101 stands by in a state to wait for reception of the access point (AP) switching preparation completion notification.

On the other hand, if the radiation imaging apparatus 101 determines in step SX406 that the access point (AP) switching preparation completion notification is received (YES in step SX406), the process advances to step SX407. In step SX407, the radiation imaging apparatus 101 stops the AP mode operation and controls the display on the display unit to notify the radiographer as the user that the AP mode has stopped. In step SX408, the radiation imaging apparatus 101 ends the AP mode. The processing of the console and the radiation imaging apparatus when the wireless communication operation is unstable has been described above.

Processing in a case in which wireless communication between the radiation imaging apparatus 101 operating as an access point (AP) and another device cannot stably be performed has exemplarily been described with reference to Figs. 4A and 4B. However, this embodiment is not limited to this example. For example, the same processing as that described with reference to Figs. 4A and 4B can be executed when an operation state in which the remaining capacity of the internal power supply 207 of the radiation imaging apparatus 101 is smaller than a threshold, and the radiation imaging apparatus 101 needs to stop the function after the elapse of a predetermined time is revealed. In this case, for example, each of the processes of steps SX403 and SC405 in Figs. 4A and 4B is replaced with processing of determining whether the remaining capacity of the internal battery is equal to or larger than a threshold, thereby allowing the console and the radiation imaging apparatus to execute the same processing as described above.

Independently of this embodiment, processing of switching the operation of the radiation imaging apparatus 101 to the slave unit in a case in which information exchange by wireless communication is not executed between the console 103 and the radiation imaging apparatus 101 operating as an access point (AP) for a predetermined time can be executed by replacing part of the processing shown in Figs. 4A and 4B. More specifically, the time in which information exchange by wireless communication is not executed is defined as "communication absent time". Each of the processes of steps SC405 and SX403 in Figs. 4A and 4B is changed to "communication absent time ≥ set time?". This makes it possible to switch the operation of the radiation imaging apparatus 101 from the AP mode in which the radiation imaging apparatus operates as a master unit to the slave unit mode (STA mode) in which the radiation imaging apparatus operates as a slave unit.

If wireless communication is not performed for a predetermined time or more, the console 103 (control apparatus) can make the radiation imaging apparatus operating as the master unit as a slave unit. The radiation imaging apparatus 101 that is performing the AP operation without wireless communication thus operates as a slave unit under the control of its own or based on an instruction from the console 103. If the radiation imaging apparatus operates as an access point (AP), the number of processes to be executed may increase as compared a case in which the radiation imaging apparatus operates as a slave unit. The power consumption may also increase along with the increase in the number of processes. As described above here, when the radiation imaging apparatus 101 is designated as a slave unit between the console 103 and the radiation imaging apparatus 101 which do not perform wireless communication, the power consumption can be suppressed.

The processing described with reference to Figs. 4A and 4B is triggered by the processing of the console 103 or the radiation imaging apparatus 101. However, it may be triggered by access point (AP) switching or the AP mode stop operation based on an operation of the radiographer. For example, when switching a device made to operate as an access point (AP) between the AP unit 104 and the radiation imaging apparatus 101, the device made to operate as an access point (AP) can be switched based on the operation of the radiographer.

### (Modification)

In the first embodiment, the operations of the radiation imaging apparatus and the console (control apparatus) in one radiation imaging system have been described. However, the present invention is not limited to this example, and is also applicable to a case in which, for example, wireless communication enable regions overlap in a plurality of radiation imaging systems.

For example, in a case in which a first radiation imaging system and a second radiation imaging system each including a radiation imaging apparatus and a control apparatus exist in adjacent imaging rooms or the like of a hospital, regions where wireless communication is possible overlap, and the control apparatus of the first radiation imaging system can wirelessly be connected to the radiation imaging apparatus of the second radiation imaging system. In this case, a transmission error may occur if the control apparatus of the first radiation imaging system generates the same wireless identification information (for example, SSID) as that of the radiation imaging apparatus of the first radiation imaging system for the radiation imaging apparatus of the second radiation imaging system other than the first radiation imaging system, which is actually going to do imaging.

To prevent the transmission error, the radiation imaging system according to this modification includes a control apparatus capable of performing wireless communication with a radiation imaging apparatus that can operate as a master unit or slave unit in wireless communication. The control apparatus includes a determination unit configured to determine whether a radiation imaging apparatus capable of performing wireless communication exists in the radiation imaging system, and a generation unit configured to, if a plurality of radiation imaging apparatuses capable of performing wireless communication exist based on the determination of the determination unit, generate different pieces of wireless identification information for the radiation imaging apparatuses. For example, when generating wireless identification information, the generation unit can acquire wireless identification information assigned to another radiation imaging apparatus, generate wireless identification information different from the acquired wireless identification information, and set the wireless identification information for the radiation imaging apparatus of the transmission destination. The generation unit of the control apparatus performs control to give wireless identification information different from wireless identification information assigned to another radiation imaging apparatus, thereby generating different pieces of wireless identification information for the radiation imaging apparatuses even in a case in which a plurality of radiation imaging apparatuses capable of performing wireless communication exist. It is therefore possible to prevent a transmission error.

### (Second Embodiment)

In this embodiment, a case in which a second radiation imaging apparatus is newly added to a radiation imaging system in which one console 103 and one radiation imaging apparatus 101 exist, as in the above-described first embodiment, will be described. As for the arrangement, one radiation imaging apparatus is newly added to the arrangement shown in Fig. 2.

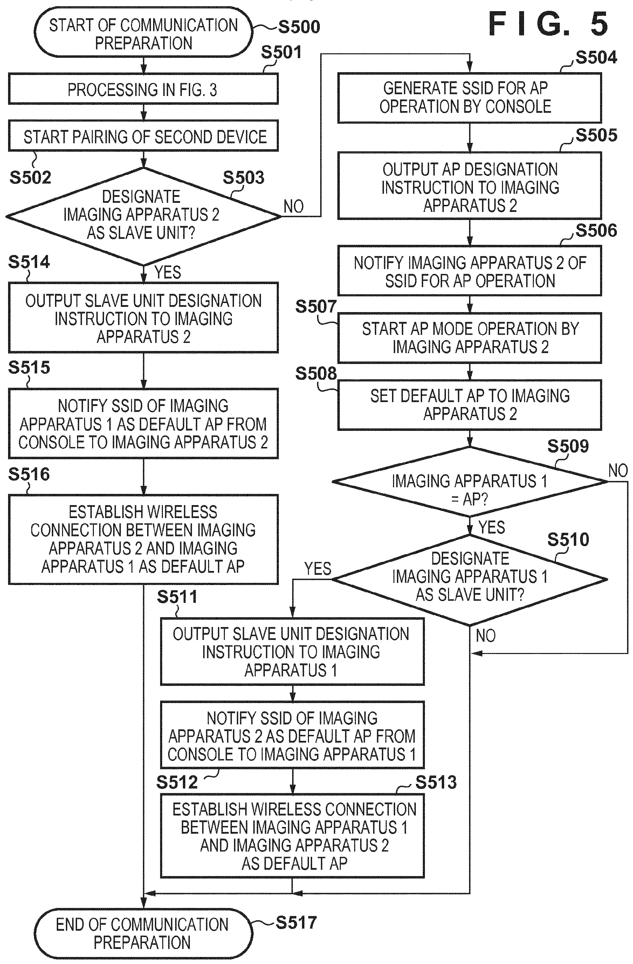

Fig. 5 is a flowchart for explaining communication preparation processing when using a plurality of radiation imaging apparatuses. The procedure of processing when performing pairing of the second radiation imaging apparatus will be described below with reference to Fig. 5. In this processing, if a radiation imaging apparatus is newly added to the radiation imaging system in which a radiation imaging apparatus is operating as the master unit of wireless communication, the console 103 (control apparatus) selects whether to make the added radiation imaging apparatus operate as a slave unit or master unit of wireless communication. The console 103 (control apparatus) then controls the operation of the added radiation imaging apparatus based on the selection.

The contents of processing after communication preparation starts until pairing of the first radiation imaging apparatus (to be referred to as an imaging apparatus 1 hereinafter) is performed, and communication preparation is completed are the same as in Fig. 3. For this reason, in Fig. 5, the communication preparation processing of the imaging apparatus 1 in step S501 will be explained as processing of Fig. 3, and a detailed description thereof will be omitted. Note that as the result of the process of step S501, one of an AP unit 104 and the imaging apparatus 1 is set to the default connection destination AP. In this embodiment, the imaging apparatus 1 is set to the default connection destination AP.

When pairing of the second radiation imaging apparatus (to be referred to as an imaging apparatus 2 hereinafter) starts in step S502, the console 103 determines in step S503 whether to use the imaging apparatus 2 as a slave unit. The determination processing of step S503 can be executed by the same processing as the processing (step S309 in Fig. 3) of determining whether to use the imaging apparatus 1 as a slave unit.

If the radiographer as the user selects not to use the imaging apparatus 2 as a slave unit, that is, to make the imaging apparatus 2 operate as an access point (AP: master unit) (NO in step S503), the process advances to step S504. When the operation of the master unit is selected, the console 103 (control apparatus) makes the radiation imaging apparatus operating as the master unit operate as a slave unit and then makes the added radiation imaging apparatus operate as the master unit of wireless communication based on unique wireless identification information.

Processing (steps S504 to S508) when using the imaging apparatus 2 as an access point (AP: master unit) is almost the same as the processing (SU300 in Fig. 3) when making the imaging apparatus 1 operate as an access point (AP).

More specifically, in step S504, the console 103 generates an SSID for the AP operation as wireless identification information (wireless ID) necessary for the imaging apparatus 2 to operate in the AP mode. In this system, since a plurality of radiation imaging apparatuses (imaging apparatuses 1 and 2) are incorporated in the system, the console 103 generates an SSID as unique wireless identification information (wireless ID).

When a unique SSID is generated in step S504, the process advances to step S505. In step S505, the console 103 outputs an instruction (AP designation instruction) to the imaging apparatus 2 to make it operate in the AP mode.

In step S506, the console 103 notifies the imaging apparatus 2 of the SSID generated in step S504. In step S507, the imaging apparatus 2 that has received the instruction and the SSID information from the console 103 starts an operation (AP mode operation) as an access point (AP) using the received SSID. After that, in step S508, wireless connection between the console 103 and the imaging apparatus 2 in the AP mode is established. When a predetermined time elapses from the SSID notification or the console 103 receives a preparation completion notification from the imaging apparatus 2 that has started the operation in the AP mode, wireless connection between the console 103 and the imaging apparatus 2 notified of the SSID is established. The console 103 changes the access point (AP) as the default connection destination of itself from the imaging apparatus 1 to the imaging apparatus 2. By this processing, the imaging apparatus 2 is set as the default access point (AP).

Next, in step S509, the console 103 determines whether the imaging apparatus 1 is operating as an access point (AP). If the imaging apparatus 1 is not operating as an access point (AP) (NO in step S509), the process advances to step S517 to complete communication preparation.

On the other hand, upon determining in step S509 that the imaging apparatus 1 is operating as an access point (AP) (YES in step S509), the process advances to step S510. When the process advances to step S510, the console 103 determines whether to designate the imaging apparatus 1 as a slave unit.

In this embodiment, since each of the imaging apparatus 1 and the imaging apparatus 2 has a unique SSID, both imaging apparatuses can simultaneously operate as access points (APs). However, in some cases, an imaging apparatus that need not operate as an access point (AP) is preferably switched to the operation as a slave unit from the viewpoint of power saving or in order to release limited wireless channels as much as possible. Hence, in this step, it is determined whether to switch the imaging apparatus 1 operating as an access point (AP) to the operation as a slave unit.

The console 103 displays, on the monitor, a window to confirm with the user about the manner the paired imaging apparatus 1 is made to operate, and prompts the radiographer as the user to make selection. If making the imaging apparatus 1 operate as an access point (AP: master unit) is selected as the result of selection of the radiographer (NO in step S510), the process advances to step S517 to end the processing. On the other hand, if making the imaging apparatus 1 operate as a slave unit is selected by the radiographer in the determination of step S510, the process advances to step S511.

When the process advances to step S511, the console 103 notifies the imaging apparatus 1 of an instruction (slave unit designation instruction) to make it operate as a slave unit.

In step S512, the console 103 notifies the imaging apparatus 1 of the SSID of the imaging apparatus 2 that is the default access point (AP) as an access point (AP) to be connected. In step S513, wireless connection is established between the imaging apparatus 1 and the imaging apparatus 2 operating as the default access point (AP), thus completing the communication preparation (step S517).

On the other hand, if the radiographer selects making the imaging apparatus 2 operate as a slave unit as the result of determination in step S503 (YES in step S503), the process advances to step S514. When the operation of a slave unit is selected, the console 103 (control apparatus) notifies the added radiation imaging apparatus of the wireless identification information of the radiation imaging apparatus operating as a master unit, and makes the added radiation imaging apparatus operate as a slave unit of wireless communication.

When the process advances to step S514, the console 103 notifies the imaging apparatus 2 of an instruction (slave unit designation instruction) to make it operate as a slave unit. In step S515, the console 103 notifies the imaging apparatus 2 of the SSID of the imaging apparatus 1 operating as the default access point (default AP) as an access point (AP) to be connected. In step S516, wireless connection is established between the imaging apparatus 2 and the imaging apparatus 1 operating as the default access point (default AP), thus completing the communication preparation (step S517).

Processing when adding the second radiation imaging apparatus to the radiation imaging system has been described above. By executing the same processing, third and subsequent radiation imaging apparatuses can be added to the radiation imaging system.

Additionally, in the processing shown in Fig. 5, processing of designating a radiation imaging apparatus (for example, imaging apparatus 1) other than the newly added radiation imaging apparatus (imaging apparatus 2) as a slave unit is performed in steps S509 to S513. However, this embodiment is not limited to this example. For example, if pairing of a number of radiation imaging apparatuses is performed, the console 103 can select which imaging apparatus should be designated as an AP and which imaging apparatus should be designated as a slave unit. It is therefore possible to form a radiation imaging system such that a plurality of access points (APs) and a plurality of slave units coexist. In the processing shown in Fig. 5, the console can also include a selection unit configured to allow the radiographer as the user to select the AP designation or slave unit designation at an arbitrary timing.

### (Example of Processing of Console and Radiation Imaging Apparatus When Wireless Communication Operation Is Unstable)

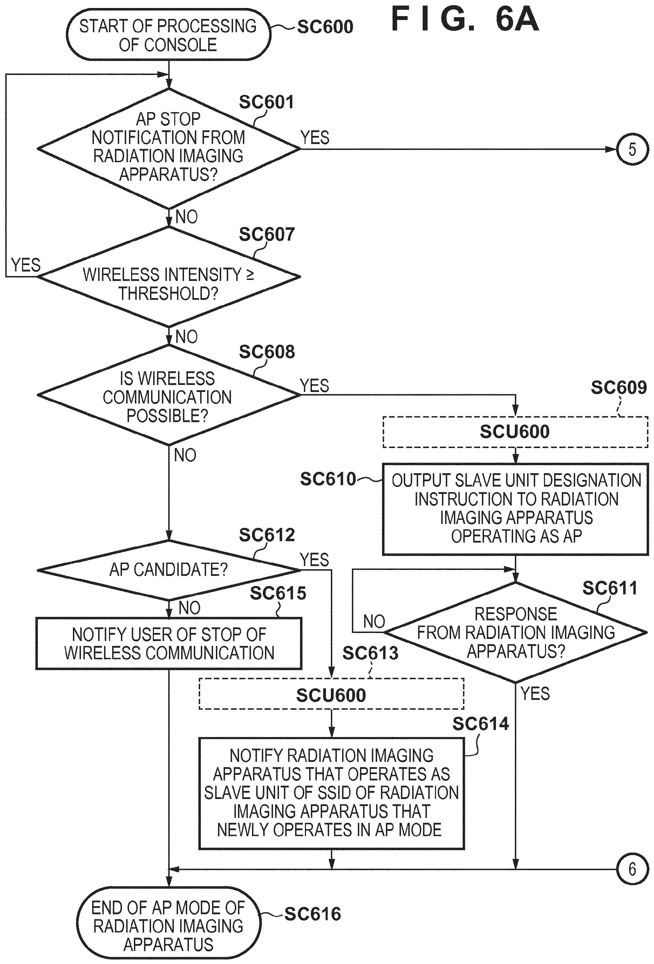

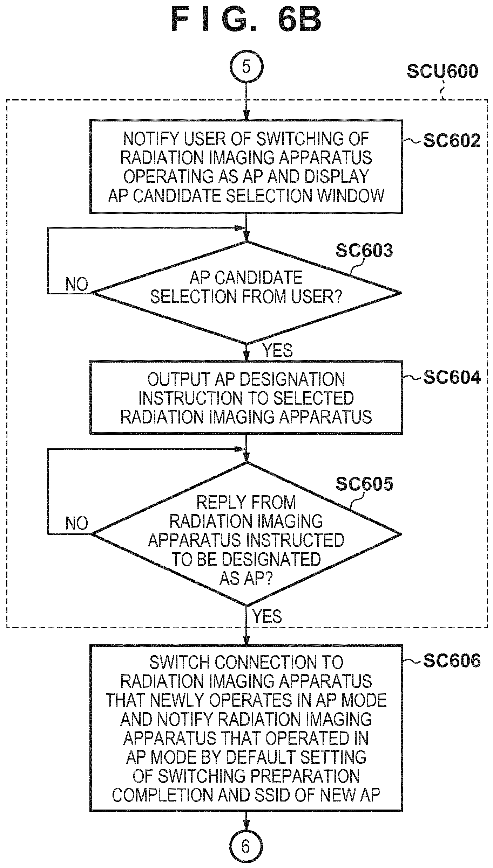

Processing of the radiation imaging apparatus and the console in a case in which the radiation imaging apparatus is carried to a place apart from the system, or the operation becomes unstable when the radiation imaging apparatus is operating as an access point (AP) will be described next with reference to Figs. 6A and 6B.

Figs. 6A and 6B correspond to the processing on the side of the console 103 out of the processing shown in Figs. 4A and 4B described above. Processing of the console 103 in a case in which a plurality of radiation imaging apparatuses exist in the radiation imaging system is added. A step number in the flowchart is indicated by "SC".

Processing (SC600) of the console 103 will be described below in detail. In step SC601, if the radiation imaging apparatus 101 operating in the AP mode exists in the radiation imaging system, the console 103 monitors the presence/absence of a notification (AP stop notification) to stop the operation as an access point (AP) from the radiation imaging apparatus 101.

Upon receiving the notification (AP stop notification) from the radiation imaging apparatus 101 (YES in step SC601), the console 103 advances the process to step SC602.

In step SC602, the console 103 notifies the radiographer as the user that the radiation imaging apparatus operating as an access point (AP) needs to be switched, and also notifies the radiographer of a radiation imaging apparatus capable of operating operate as an access point (AP) and the AP unit 104 as candidates. The console 103 can make the radiation imaging apparatus switching notification by, for example, displaying the access point (AP) candidates on the display unit such as a monitor (AP candidate selection screen display). For example, a radiation imaging apparatus capable of operating as an access point (AP) is assigned a unique SSID at the time of pairing for the processing. It is therefore possible to display all radiation imaging apparatuses existing in the system on the AP candidate selection screen as the candidates.

In step SC603, the console 103 determines the presence/absence of access point (AP) candidate selection from the user. That is, the console 103 determines whether an instruction of a radiation imaging apparatus to be made to operate as an access point (AP) is given by the radiographer as the user.

If a selection instruction from the user does not exist (NO in step SC603), the console 103 stands by in a state to wait for a selection instruction from the user. On the other hand, upon determining in step SC603 that a selection instruction from the user exists (YES in step SC603), the process advances to step SC604.

In step SC604, the console 103 outputs an instruction (AP designation instruction) to the selected target radiation imaging apparatus to make it operate in the AP mode.

After that, when the process advances to step SC605, the console 103 determines the presence/absence of a response (a response of notification reception completion or AP designation completion) from the radiation imaging apparatus (AP candidate imaging apparatus) instructed to be designated as an AP. If a response from the radiation imaging apparatus (AP candidate imaging apparatus) instructed to be designated as an AP does not exist (NO in step SC605), the console 103 stands by in a state to wait for a response from the radiation imaging apparatus. On the other hand, upon determining in step SC605 that a response from the radiation imaging apparatus (AP candidate imaging apparatus) exists (YES in step SC605), the process advances to step SC606.

When the process advances to step SC606, the console 103 switches the connection to the radiation imaging apparatus (for example, imaging apparatus 2) that newly operates as an access point (AP). The console 103 then notifies the radiation imaging apparatus 101 operating as an access point (AP) by default setting that access point (AP) switching preparation is completed. The console 103 also notifies the radiation imaging apparatus 101 of the SSID of the radiation imaging apparatus (imaging apparatus 2) that newly operates as an access point (AP). That is, the console 103 switches the connection to the radiation imaging apparatus (imaging apparatus 2) that newly operates as an access point (AP), and notifies the radiation imaging apparatus 101 operating as an access point (AP) by default setting of the switching preparation completion and the SSID of the new AP (imaging apparatus 2).

After step SC606, the process advances to step SC616. In step SC616, the console 103 ends the AP mode of the radiation imaging apparatus 101. The AP mode operation of the radiation imaging apparatus 101 operating as an access point (AP) ends.

On the other hand, upon determining in step SC601 that the notification (AP stop notification) from the radiation imaging apparatus 101 operating in the AP mode is not received (NO in step SC601), the console 103 advances the process to step SC607.

In step SC607, the console 103 confirms the wireless intensity (wireless sensitivity) between the console 103 and the radiation imaging apparatus 101 that is the connection destination operating in the AP mode. Upon determining that the wireless intensity (wireless sensitivity) is equal to or higher than a threshold, and wireless communication can stably be performed (YES in step SC607), the console 103 returns the process to step SC601 to repeat the same processing. On the other hand, if the wireless intensity (wireless sensitivity) is lower than the threshold in step SC607 (NO in step SC607), the process advances to step SC608.

In step SC608, if the console 103 determines that wireless communication is possible (YES in step SC608), the process advances to step SC609. The processing contents of step SC609 are the same as those of steps SC602 to SC605 described above. In Fig. 6B, these processes are defined as SCU600 that replaces the description in step SC609. When preparation of the radiation imaging apparatus (AP candidate imaging apparatus) as a new AP candidate is completed by the process of SCU600 in step SC609, the process advances to step SC610.

In step SC610, the console 103 notifies the radiation imaging apparatus 101 operating as an access point (AP) of an instruction (slave unit designation instruction) to do an operation as a slave unit.

In step SC611, the console 103 determines whether a response to the slave unit designation instruction notification is received from the radiation imaging apparatus 101. If a response to the slave unit designation instruction notification is not received (NO in step SC611), the console 103 stands by in a state to wait for a response. On the other hand, if a response to the slave unit designation instruction notification is received (YES in step SC611), the process advances to step SC616. In step SC616, the console 103 ends the AP mode of the radiation imaging apparatus 101. The AP mode operation of the radiation imaging apparatus 101 operating as an access point (AP) ends.

On the other hand, if the console 103 determines in step SC608 that wireless communication is impossible (NO in step SC608), the process advances to step SC612.

In step SC612, the console 103 determines whether a radiation imaging apparatus capable of operating as an access point (AP) or the AP unit 104 exists in the radiation imaging system as an access point (AP) candidate (AP candidate). For example, the same processing as the processing of determining whether the AP unit 104 exists in the radiation imaging system, like the process of step S303 in Fig. 3 or the process of step SC410 in Fig. 4A, can be applied to the determination processing of step SC612.

Upon determining in step SC612 that an access point (AP) candidate (AP candidate) exists in the radiation imaging system (YES in step SC612), the process advances to step SC613.

The processing contents of step SC613 are the same as those of steps SC602 to SC605 described above. In Fig. 6B, these processes are defined as SCU600 that replaces the description in step SC613. When preparation of the radiation imaging apparatus (AP candidate imaging apparatus) as a new AP candidate is completed by the process of SCU600 in step SC613, the process advances to step SC614.

In step SC614, the console 103 notifies the radiation imaging apparatus 101 operating as an access point (AP) of an instruction (slave unit designation instruction) to do an operation as a slave unit. The console 103 then notifies the radiation imaging apparatus 101 that operates as a slave unit of the SSID of the radiation imaging apparatus that newly operates in the AP mode.

That is, the console 103 notifies the wireless slave unit connected to the original access point (AP) of the information (AP information) of the new access point (AP). At this time, since the radiation imaging apparatus that originally operated as an access point (AP) cannot be connected to wireless communication, the console 103 notifies the radiation imaging apparatus that originally operated as an access point (AP) of the SSID of the new access point (AP) that newly operates in the AP mode without intervention of the conventional access point (AP). Several methods of notifying the SSID of the new access point (AP) are assumed.

For example, the console 103 acquires the information of a device connected as a slave unit in advance from the radiation imaging apparatus operating as an access point (AP). If the access point (AP) is absent, the console 103 can notify the SSID of the new access point (AP) based on the acquired information of the slave unit.

Alternatively, instead of using the SSID assigned at the time of pairing, setting may be done such that the console 103 generates an SSID including the information of the SSID of the access point (AP) that originally operated or an SSID including information representing an access point (AP) to be preferentially connected such that the slave unit connects with the access point (AP) of the new SSID at its own discretion.

For example, assume that the SSID of the radiation imaging apparatus operated as an access point (AP) is "AP_ID1_YYMMDD1". This information is known in the console 103.

When instructing another radiation imaging apparatus to newly operate in the AP mode (AP designation instruction), the console 103 generates an SSID by adding the known SSID information "AP_ID1_YYMMDD1" to the start of an SSID to be assigned to another radiation imaging apparatus. For example, if the SSID of the radiation imaging apparatus that newly operates as an access point (AP) is "AP_ID2_YYMMDD2", the console 103 generates "AP_ID1_YYMMDD1_AP_ID2_YYMMDD2" as the SSID of another radiation imaging apparatus that newly operates as an access point (AP). The slave unit connected to the original access point (AP) can be determined, based on the name of the SSID, to be a master unit that is prepared as a replacement and newly operates in the AP mode.

The console 103 can also preset additional information (for example, a character string) such as "1stPri_AP" to be added to the SSID of an access point (AP) to be preferentially connected. When switching the access point (AP), the console 103 can add additional information representing the priority order of connection to the SSID of the master unit that newly operates in the AP mode. When such an SSID is set, the console 103 may shift to the originally assigned SSID for the AP operation after a predetermined time. When switching the access point (AP), the console 103 can notify the slave unit connected to the access point (AP) of the switching of the SSID such that connection of the slave unit to the new access point (AP) after the switching can be reestablished.

After step SC614, the process advances to step SC616. In step SC616, the console 103 ends the AP mode of the radiation imaging apparatus 101. The AP mode operation of the radiation imaging apparatus 101 operating as an access point (AP) ends.

On the other hand, upon determining in step SC612 that an access point (AP) candidate (AP candidate) does not exist in the radiation imaging system (NO in step SC612), the process advances to step SC615.

In step SC615, if an access point (AP) candidate (AP candidate) does not exist in the radiation imaging system, the console 103 notifies the radiographer as the user, via the monitor of the console 103 or the like, that wireless communication stops. The console 103 then ends the AP mode of the radiation imaging apparatus 101. The AP mode operation of the radiation imaging apparatus 101 operating as an access point (AP) ends.

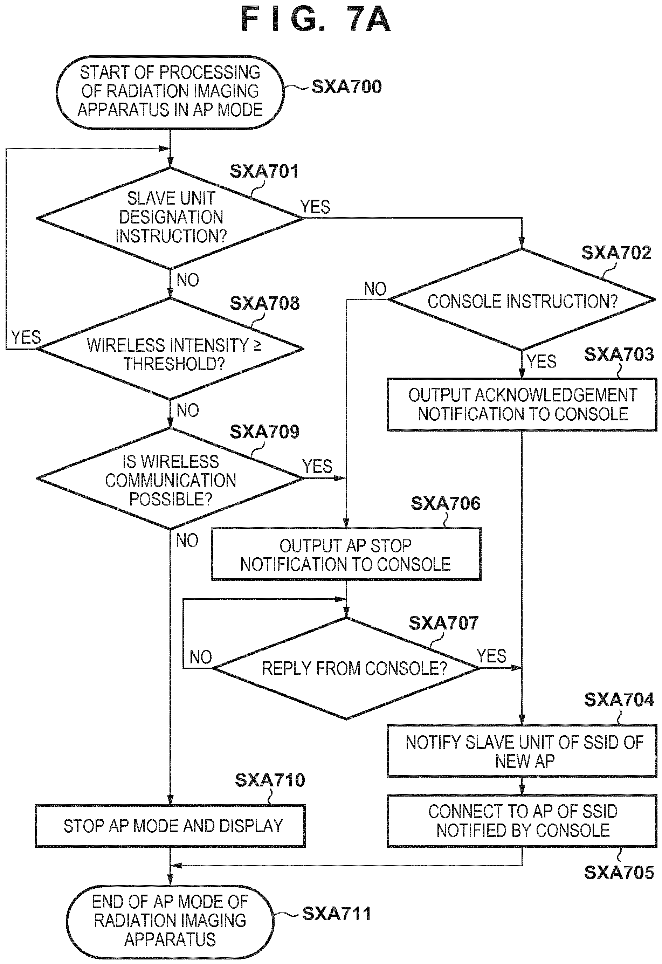

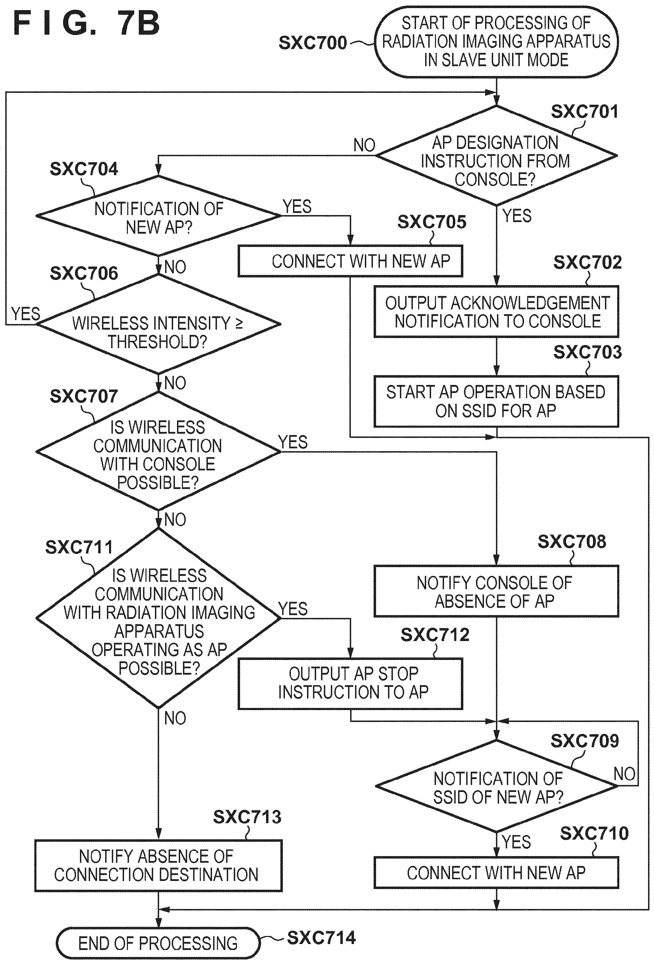

Processing on the radiation imaging apparatus side that is the counterpart of the processing on the console side shown in Figs. 6A and 6B will be described next with reference to Figs. 7A and 7B. Figs. 7A and 7B show the processing of a radiation imaging apparatus that is performing the AP operation and a radiation imaging apparatus that is performing the slave unit operation.

Fig. 7A illustrates the processing of a radiation imaging apparatus (to be referred to as the radiation imaging apparatus 101 hereinafter) operating as an access point (AP), and Fig. 7B illustrates the processing of a radiation imaging apparatus (to be referred to as a radiation imaging apparatus 151 hereinafter) operating as a slave unit. A step number in the flowchart of Fig. 7A is indicated by "SXA", and a step number in the flowchart of Fig. 7B is indicated by "SXC".

### (Processing of Radiation Imaging Apparatus Operating in AP Mode)

The processing of the radiation imaging apparatus 101 operating as an access point (AP) (operating in the AP mode) will be described first with reference to Fig. 7A. In step SXA700, the processing of the radiation imaging apparatus 101 operating in the AP mode starts. In step SXA701, the radiation imaging apparatus 101 determines the presence/absence of a slave unit designation instruction notification. If the radiation imaging apparatus 101 determines in step SXA701 that a slave unit designation instruction notification is received (YES in step SXA701), the process advances to step SXA702.

In step SXA702, the radiation imaging apparatus 101 determines whether the transmission source of the received slave unit designation instruction notification is the console 103. If the transmission source is the console 103 (YES in step SXA702), the process returns to step SXA703. The console 103 as the transmission source notifies the SSID of the master unit that newly operates as an access point (AP) together with the slave unit designation instruction.

In step SXA703, the radiation imaging apparatus 101 transmits an acknowledgement notification to the received slave unit designation instruction notification to the console 103. After step SXA703, the process advances to step SXA704.

On the other hand, upon determining in step SXA702 that the transmission source of the received slave unit designation instruction is not the console 103 but, for example, a slave unit of wireless communication (NO in step SXA702), the process advances to step SXA706. In step SXA706, the radiation imaging apparatus 101 transmits a notification (AP stop notification) to stop the operation as an access point (AP) to the console 103.

In step SXA707, the radiation imaging apparatus 101 determines the presence/absence of an access point (AP) switching preparation completion notification (the notification transmitted in step SC606 of Fig. 6B) transmitted from the console 103 as a reply to the transmitted AP stop notification.

Upon determining in step SXA707 that the access point (AP) switching preparation completion notification is not received (NO in step SXA707), the radiation imaging apparatus 101 stands by in a state to wait for reception of the access point (AP) switching preparation completion notification.

On the other hand, if the radiation imaging apparatus 101 determines in step SXA707 that the access point (AP) switching preparation completion notification is received (YES in step SXA707), the process advances to step SXA704. The console 103 notifies the SSID of the master unit that newly operates as an access point (AP) together with the switching preparation completion notification.

In step SXA704, the radiation imaging apparatus 101 notifies the slave unit connected to itself of the SSID of the master unit (new AP) that newly operates as an access point (AP). In step SXA705, the radiation imaging apparatus 101 is connected to the access point (AP) of the SSID notified by the console 103. In step SXA711, the radiation imaging apparatus 101 ends the AP mode operation.

On the other hand, if the radiation imaging apparatus 101 determines in step SXA701 that a slave unit designation instruction notification is not received (NO in step SXA701), the process advances to step SXA708.

In step SXA708, the radiation imaging apparatus 101 confirms the wireless intensity (wireless sensitivity). Upon determining that the wireless intensity (wireless sensitivity) is equal to or higher than a threshold, and wireless communication can stably be performed (YES in step SXA708), the radiation imaging apparatus 101 returns the process to step SXA701 to repeat the same processing. On the other hand, if the wireless intensity (wireless sensitivity) is lower than the threshold in step SXA708, for example, if the radiation imaging apparatus 101 determines that a stable wireless intensity (wireless sensitivity) cannot be ensured (NO in step SXA708), the process advances to step SXA709. The threshold used here can be set to a lower limit intensity (lower limit sensitivity) at which wireless communication is possible or a value with a margin to some extent with respect to the lower limit intensity (lower limit sensitivity).

In step SXA709, the radiation imaging apparatus 101 determines whether wireless communication is possible. As described above, even in a case in which the wireless intensity is equal to or lower than the threshold, if the threshold is a numerical value including a margin, communication may be continued, although it may become unstable. If the radiation imaging apparatus 101 determines in step SXA709 that wireless communication is possible (YES in step SXA709), the process advances to step SXA706. As processing from step SXA706, the above-described processing is executed.

On the other hand, if the radiation imaging apparatus 101 determines in step SXA709 that wireless communication is impossible (NO in step SXA709), the process advances to step SXA710. In step SXA710, the radiation imaging apparatus 101 stops the AP mode operation, and controls the display on the display unit to notify the radiographer as the user that the AP mode has stopped. In step SXA711, the radiation imaging apparatus 101 ends the AP mode.

### (Processing of Radiation Imaging Apparatus Operating as Slave unit)

The processing of the radiation imaging apparatus 151 operating in the slave unit mode (STA mode) will be described next with reference to Fig. 7B. The internal arrangement of the radiation imaging apparatus 151 is the same as the example of the internal arrangement of the radiation imaging apparatus 101 described with reference to Fig. 2.

In step SXC700, the processing of the radiation imaging apparatus 151 operating in the slave unit mode starts. In step SXC701, the radiation imaging apparatus 151 determines whether an instruction (AP designation instruction) to make it operate in the AP mode is received from the console 103. Upon determining in step SXC701 that an AP designation instruction is received (YES in step SXC701), the process advances to step SXC702. In step SXC702, the radiation imaging apparatus 151 transmits an acknowledgement notification to the received AP designation instruction to the console 103. In step SXC703, the radiation imaging apparatus 151 starts the operation in the AP mode based on an SSID (AP SSID) used to operate in the AP, which is transmitted from the console 103. In step SXC714, the processing of changing the operation mode of the radiation imaging apparatus 151 from the slave unit mode (STA mode) to the AP mode ends.

On the other hand, upon determining in step SXC701 that an AP designation instruction is not received (NO in step SXC701), the process advances to step SXC704.

In step SXC704, the radiation imaging apparatus 151 determines whether a master unit (new AP) that newly operates as an access point (AP) different from the currently connected access point (AP) is notified by the console 103. If a master unit (new AP) that newly operates as an access point (AP) is notified by the console 103 (YES in step SXC704), the process advances to step SXC705. In step SXC705, the radiation imaging apparatus 151 is connected to the access point (new AP) of the SSID notified by the console 103. In step SXC714, the radiation imaging apparatus 151 ends the processing of changing the access point (AP) as the connection destination.

If a master unit (new AP) that newly operates as an access point (AP) is not notified by the console 103 in step SXC704 (NO in step SXC704), the process advances to step SXC706. In step SXC706, the radiation imaging apparatus 151 operating in the slave unit mode (STA mode) confirms the wireless intensity (wireless sensitivity). Upon determining that the wireless intensity (wireless sensitivity) is equal to or higher than a threshold, and wireless communication can stably be performed (YES in step SXC706), the radiation imaging apparatus 151 returns the process to step SXC701 to repeat the same processing.

On the other hand, if the wireless intensity (wireless sensitivity) is lower than the threshold in step SXC706, the process advances to step SXC707. In step SXC707, the radiation imaging apparatus 151 determines whether wireless communication with the console 103 is possible. When the access point (AP) is normally operating, the radiation imaging apparatus 151 acquires and saves the communication information of the console 103, thereby executing communication with the console 103.

If the radiation imaging apparatus 151 determines in step SXC707 that wireless communication is possible (YES in step SXC707), the process advances to step SXC708.

In step SXC708, the radiation imaging apparatus 151 notifies the console 103 that the access point (AP) is absent, and the process advances to step SXC709.

In step SXC709, the radiation imaging apparatus 151 determines whether the SSID of a master unit (new AP) that newly operates as an access point (AP) is notified by the console 103. If the SSID of a master unit (new AP) is not notified by the console 103 (NO in step SXC709), the radiation imaging apparatus 151 stands by in a state to wait for the SSID notification. On the other hand, upon determining in step SXC709 that the SSID of a master unit (new AP) is notified by the console 103 (YES in step SXC709), the process advances to step SXC710.

In step SXC710, the radiation imaging apparatus 151 is connected to the master unit (new AP) that newly operates as an access point (AP) based on the SSID transmitted from the console 103, and ends the processing (step SXC714).

If the radiation imaging apparatus 151 determines in step SXC707 that wireless communication is impossible (NO in step SXC707), the process advances to step SXC711.

In step SXC711, the radiation imaging apparatus 151 determines whether communication with the radiation imaging apparatus operating as an access point (AP) is possible. If communication with the radiation imaging apparatus operating as an access point (AP) is possible as the determination result (YES in step SXC711), the process advances to step SXC712. In step SXC712, the radiation imaging apparatus 151 instructs the radiation imaging apparatus operating as an access point (AP) to stop the operation in the AP mode (AP stop instruction). After the process of step SXC712, the process advances to step SXC709. As processing from step SXC709, the above-described processing is executed.

On the other hand, if communication with the radiation imaging apparatus operating as an access point (AP) is impossible in step SXC711 (NO in step SXC711), the process advances to step SXC713. The radiation imaging apparatus 151 operating in the slave unit mode (STA mode) notifies, via the display unit or the like, that no connection destination is available as a wireless slave unit, and ends the processing (step SXC714) .

In this embodiment, a case in which devices such as the console 103, the AP unit 104, and the radiation imaging apparatuses coexist in the radiation imaging system has been described. The system may include a device other than those described above. For example, if the radiographer as the user wants to do an operation via a tablet PC that is superior in portability but inferior in processing performance to the console 103, a system arrangement in which the tablet PC is connected to the console 103 via an access point (AP) so as to be remote-controllable is assumed. In such a system arrangement, the above-described arrangement and processing of the embodiment can be applied by replacing the slave unit with the tablet PC. Note that an information processing apparatus such as the above-described tablet PC can have a function of operating as an access point (AP) to itself. Having the function of operating as an access point (AP), the information processing apparatus can execute the same processing procedure as the processing of the radiation imaging apparatus. On the other hand, even without the function of operating as an access point (AP), the arrangement need only prohibit the AP designation instruction to make the apparatus operate in the AP mode from being given. Even if the AP designation instruction is given, the arrangement need only output a notification to reject it.

### (Third Embodiment)

In the second embodiment, an arrangement in which the number of units operating as access points (APs: master units) is not particularly limited has been described. However, the limit of the number of units operating as access points (APs) (for example, the upper limit of the number of units or the lower limit of the number of units, or both of them) may be set. If the number of units exceeds the set limit, the user can be notified of that situation via the display unit of a console 103 or a radiation imaging apparatus. In this embodiment, communication preparation processing, processing of adding a radiation imaging apparatus to a system, and processing of stopping a radiation imaging apparatus operating in the AP mode in a radiation imaging system that implements the above-described arrangement will be described.

### (Communication Preparation Processing)

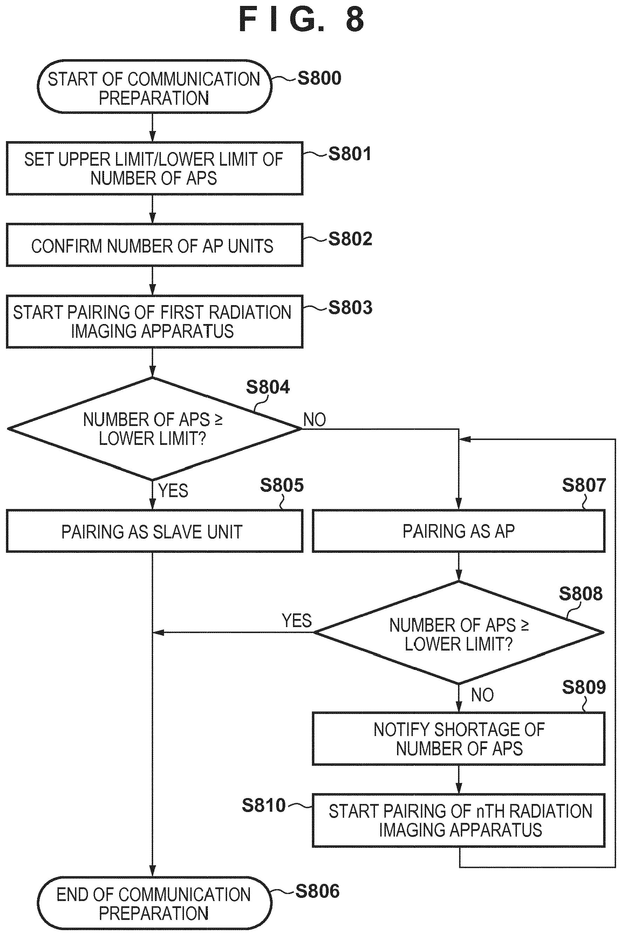

Fig. 8 is a flowchart for explaining the procedure of communication preparation processing in the console when setting the limit of the number of units that operate as access points (APs).

When starting imaging, each unit is activated, as described concerning step S301 of Fig. 3 in the first embodiment.

In step S801, the console 103 sets the limits (the upper limit of the number of units and the lower limit of the number of units) of the number of units capable of operating as an access point (AP) based on an operation input of the user. After the limits are set, in step S802, the console 103 confirms the number of AP units 104 existing in the radiation imaging system. In step S803, the console 103 executes pairing with the first radiation imaging apparatus.

In step S804, the console 103 determines whether access points (APs) in number equal to or more than the set lower limit are ensured. For example, if the radiation imaging system includes only one AP unit 104 that is operating, and the set lower limit is one, the number of access points (APs) is equal to or more than the lower limit (YES in step S804), and the process advances to step S805. In step S805, the radiation imaging apparatus that has started pairing in step S803 is set to perform a slave unit operation. By this processing, the radiation imaging apparatus is paired with the console 103 as a slave unit. After the process of step S805, the communication preparation is completed (step S806).

On the other hand, if access points (APs) in number equal to or more than the set lower limit are not ensured in step S804 (NO in step S804), the process advances to step S807. In step S807, the console 103 sets the radiation imaging apparatus that has started pairing in step S803 to operate as an access point (AP). By this processing, the radiation imaging apparatus is paired with the console 103 as an access point (AP).

In step S808, the console 103 determines whether access points (APs) in number equal to or more than the set lower limit are ensured. In this step, the console 103 determines whether the number of access points (APs) increased in step S807 is equal to or more than the set lower limit.

If the number of access points (APs) is equal to or more than the lower limit (YES in step S808), the process advances to step S806 to complete the communication preparation.

On the other hand, upon determining in step S808 that the number of access points (APs) is not equal to or more than the lower limit (NO in step S808), the process advances to step S809. In step S809, the console 103 notifies the radiographer as the user that the number of access points (APs) is short via the display unit or the like.

In step S810, the console 103 starts pairing with the nth (n ≥ 2) radiation imaging apparatus. That is, the console 103 starts pairing processing with a new radiation imaging apparatus, and returns the process to step S807. The console 103 repetitively executes the processing from step S807 until the number of access points (APs) becomes equal to or more than the lower limit.

Note that an example of communication preparation processing in which the communication preparation is not completed unless the number of access points (APs) becomes equal to or more than the set lower limit has been described with reference to Fig. 8. However, this embodiment is not limited to this example. For example, in a state in which at least one radiation imaging apparatus is paired, the console 103 may notify that the number of access points (APs) is smaller than the lower limit (step S809), complete the communication preparation, and shift to imaging processing.

### (Processing of Adding Radiation Imaging Apparatus to System)

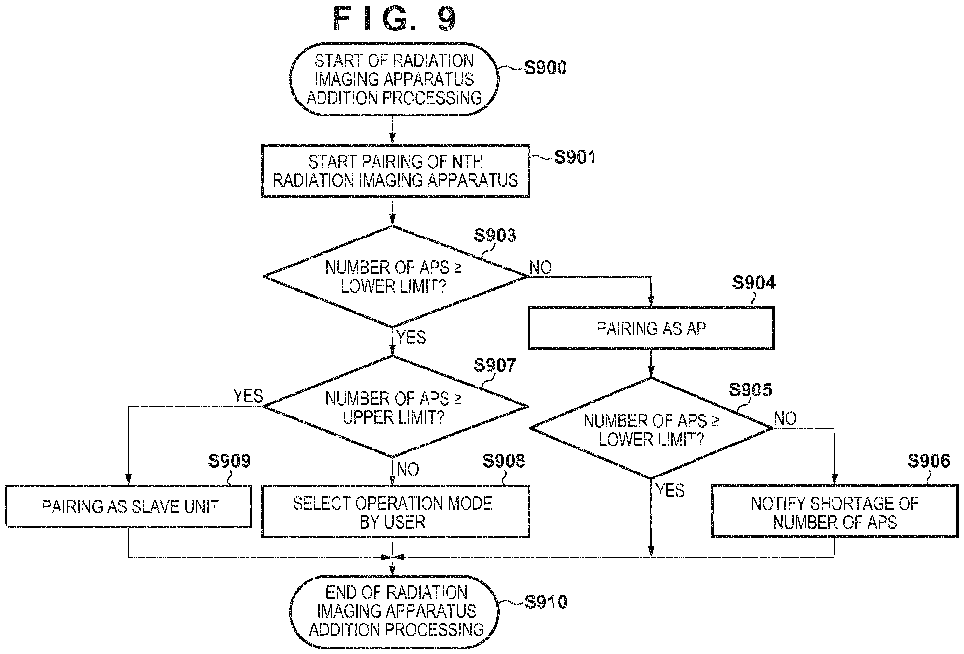

Console processing when adding a radiation imaging apparatus to the radiation imaging system will be described next with reference to Fig. 9. As described in the above embodiments, the radiation imaging apparatus can stop the operation as an access point (AP) based on a slave unit designation instruction from the console 103. Hence, when radiation imaging apparatus addition processing has started in step S900, the number of access points (APs) may be smaller than the lower limit because the operation as an access point (AP) is stopped.

In step S901, the console 103 starts pairing with the Nth (N ≥ 1) radiation imaging apparatus. That is, the console 103 starts pairing processing with a new radiation imaging apparatus, and advances the process to step S903.

In step S903, the console 103 determines whether access points (APs) in number equal to or more than the lower limit set in step S801 of Fig. 8 are ensured. If access points (APs) in number equal to or more than the set lower limit are not ensured (NO in step S903), the process advances to step S904. If the number of master units of wireless communication in the radiation imaging system is smaller than the set lower limit, the console 103 (control apparatus) makes the radiation imaging apparatus operate as a master unit of wireless communication.

In step S904, the console 103 sets the radiation imaging apparatus that has started pairing in step S901 to operate as an access point (AP). By this processing, the radiation imaging apparatus is paired with the console 103 as an access point (AP).

In step S905, the console 103 determines whether access points (APs) in number equal to or more than the set lower limit are ensured. In this step, the console 103 determines whether the number of access points (APs) increased in step S904 is equal to or more than the set lower limit. If the number of access points (APs) is equal to or more than the lower limit (YES in step S905), the process advances to step S910 to complete the radiation imaging apparatus addition processing.

If the console 103 determines in step S905 that access points (APs) in number equal to or more than the set lower limit are not ensured (NO in step S905), the process advances to step S906.

In step S906, the console 103 notifies the radiographer as the user that the number of access points (APs) is short via the display unit or the like, and the radiation imaging apparatus addition processing ends (step S910). Note that the processing from step S904 may repetitively be executed until the number of access points (APs) becomes equal to or more than the lower limit, like steps S807 to S810 of Fig. 8.

On the other hand, if the console 103 determines in step S903 that access points (APs) in number equal to or more than the lower limit set in step S801 of Fig. 8 are ensured (YES in step S903), the process advances to step S907.

In step S907, the console 103 determines whether access points (APs) in number equal to or more than the upper limit set in step S801 of Fig. 8 are ensured.

Upon determining in step S907 that the number of access points (APs) is not equal to or more than the set upper limit (NO in step S907), the process advances to step S908. In step S908, the user selects the operation mode of the radiation imaging apparatus that has started pairing, and the console 103 controls the radiation imaging apparatus based on the operation mode selection from the user to make it operate as an access point (AP) or a slave unit. If it is determined that the number of access points (APs) is less than the upper limit, the radiation imaging apparatus of the control target that has started pairing can be made to operate as an access point (AP) or a slave unit. Hence, the console 103 controls the operation of the radiation imaging apparatus of the control target based on the operation mode selection from the user. After the process of step S908, the process advances to step S910, and the radiation imaging apparatus addition processing ends.

Upon determining in step S907 that the number of access points (APs) is equal to or more than the set upper limit (YES in step S907), the process advances to step S909. In step S909, the radiation imaging apparatus that has started pairing in step S903 is set to do a slave unit operation. That is, if the number of master units of wireless communication in the radiation imaging system is equal to or more than the set upper limit, the console 103 (control apparatus) makes the radiation imaging apparatus operate as a slave unit of wireless communication. By this processing, the radiation imaging apparatus is paired with the console 103 as a slave unit. After the process of step S909, the radiation imaging apparatus addition processing ends (step S910). Note that the processes of steps S907 and S909 may repetitively be executed until the number of access points (APs) becomes less than the upper limit.

### (Processing of Stopping Radiation Imaging Apparatus Operating in AP Mode)

Processing (AP mode stop processing) of the console 103 when a radiation imaging apparatus operating in the AP mode stops the operation as an access point (AP) due to some reason will be described next.

As described in the above embodiments, the radiation imaging apparatus operating in the AP mode can stop the AP mode operation in a case in which the wireless intensity lowers or information exchange by wireless communication is not executed between the console 103 and the radiation imaging apparatus 101 operating as an access point (AP) for a predetermined time.

When the radiation imaging apparatus stops the AP mode operation, the console 103 executes this processing (step S1000). In step S1001, the console 103 determines whether access points (APs) in number equal to or more than the set lower limit are ensured even if the radiation imaging apparatus of the control target operating in the AP mode stops the AP mode operation.

If access points (APs) in number equal to or more than the set lower limit are not ensured in step S1001 (NO in step S1001), the process advances to step S1002.

In step S1002, the console 103 determines whether a radiation imaging apparatus that is operating in the slave unit mode and can operate in the AP mode or an AP unit (a unit that can be designated as an AP) exists in the radiation imaging system. To compensate for a decrease in the number of APs in the radiation imaging system caused by the stop of the AP mode operation of the radiation imaging apparatus, it is determined whether another unit that can be designated as an AP exists in the radiation imaging system. Note that in a case in which the AP mode operation is stopped due to the absence of communication between the console 103 and the radiation imaging apparatus operating as an access point (AP) for a predetermined time, processing of resuming the AP mode operation on condition that, for example, communication is performed between them can be added. In this case, a radiation imaging apparatus can be added by, for example, processing described with reference to Fig. 9.

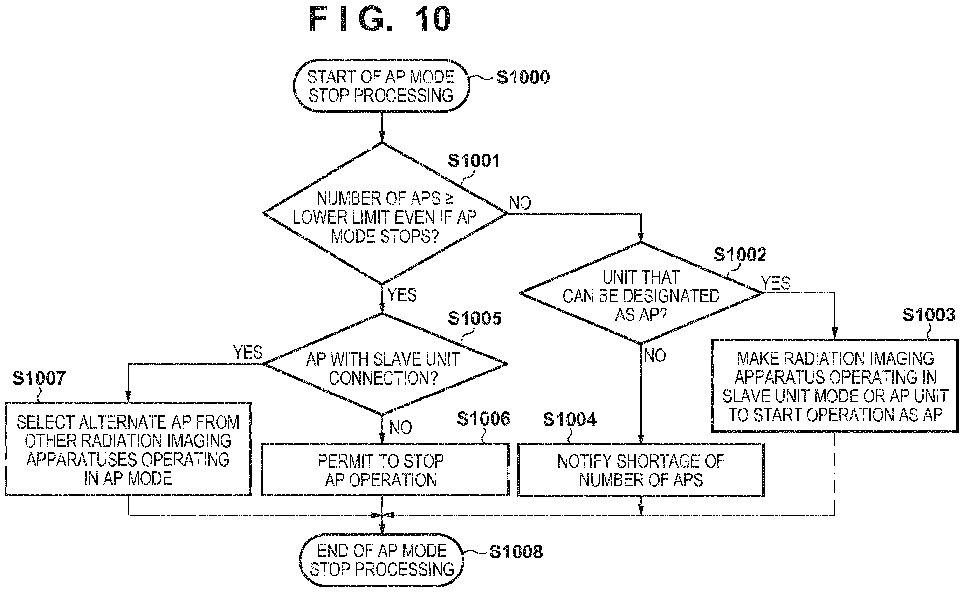

Referring back to Fig. 10, if the console 103 determines in step S1002 that a unit that can be designated as an AP exists in the radiation imaging system (YES in step S1002), the process advances to step S1003. In step S1003, the console 103 instructs the radiation imaging apparatus operating in the slave unit mode or the AP unit 104 to start an operation as an access point (AP). After the process of step S1003, the process advances to step S1008 to end the AP mode stop processing.

On the other hand, if the console 103 determines in step S1002 that a unit that can be designated as an AP does not exist in the radiation imaging system (NO in step S1002), the process advances to step S1004.

In step S1004, the console 103 notifies the radiographer as the user that the number of access points (APs) is short via the display unit or the like, and ends the AP mode stop processing (step S1008).

On the other hand, if access points (APs) in number equal to or more than the set lower limit are ensured in step S1001 (YES in step S1001), the process advances to step S1005.

In step S1005, the console 103 determines the presence/absence of slave unit connection. That is, the console 103 determines the presence/absence of a radiation imaging apparatus connected as a slave unit to the radiation imaging apparatus that stops the AP mode operation. Slave unit connection information necessary for this determination can be notified simultaneously when the radiation imaging apparatus notifies the console 103 of the stop of the AP mode operation.

Upon determining in step S1005 that slave unit connection to the radiation imaging apparatus that stops the AP mode operation is absent (NO in step S1005), the process advances to step S1006. In step S1006, the console 103 permits to stop the AP mode operation, and ends the AP mode stop processing (step S1008) .

On the other hand, upon determining in step S1005 that slave unit connection to the radiation imaging apparatus that stops the AP mode operation exists (YES in step S1005), the process advances to step S1007. In step S1007, the console 103 selects an alternate access point (AP) (alternate AP) from other radiation imaging apparatuses operating in the AP mode, and ends the AP mode stop processing (step S1008). Note that in the process of step S1001, the console 103 determines whether access points (APs) in number equal to or more than the set lower limit are ensured even if the radiation imaging apparatus operating in the AP mode stops the AP mode operation. However, there may be a radiation imaging apparatus that is operating in the AP mode but cannot be an alternate AP candidate in a case in which, for example, one access point (AP) is assigned to one system, and an operating access point (AP) is already assigned.

In this case, the console 103 can add, as an alternate AP candidate, for example, the radiation imaging apparatus operating in the slave unit mode or the AP unit 104, which has started the operation as an access point (AP), as in step S1003.

According to this embodiment, for example, in a case in which a plurality of AP units 104 and radiation imaging apparatuses operating in the AP mode exist in the radiation imaging system, even if connection to an access point (AP) set as the default connection destination is disabled due to some reason, the processing time needed to set the alternate AP can be shortened by setting the lower limit.

In addition, by setting the upper limit, an increase in the power consumption that occurs when devices more than necessary operate as access points (APs) can be suppressed while preventing the limited number of wireless channels from being unnecessarily occupied.

### (Fourth Embodiment)

In the above-described first to third embodiments, an arrangement that allows a plurality of radiation imaging apparatuses operating as access points (APs) to exist in the radiation imaging system has been described. However, the arrangement of the embodiment of the present invention is not limited to this example. An arrangement that allows only one radiation imaging apparatus to operate as an access point (AP) in the radiation imaging system is also possible.

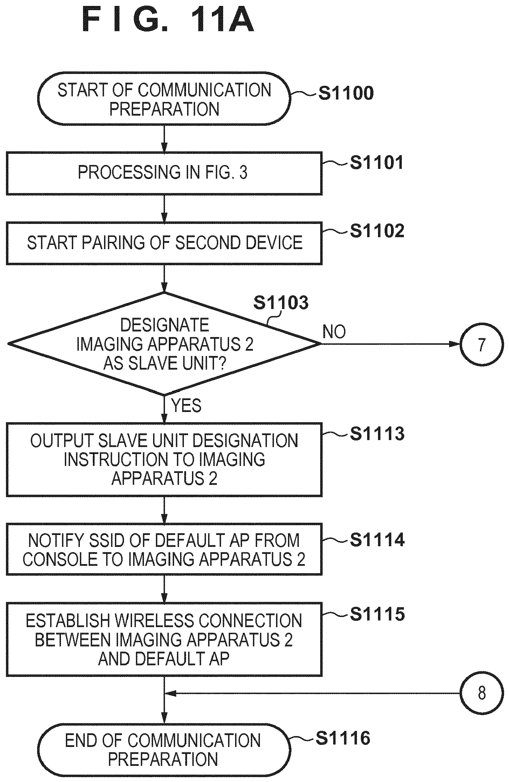

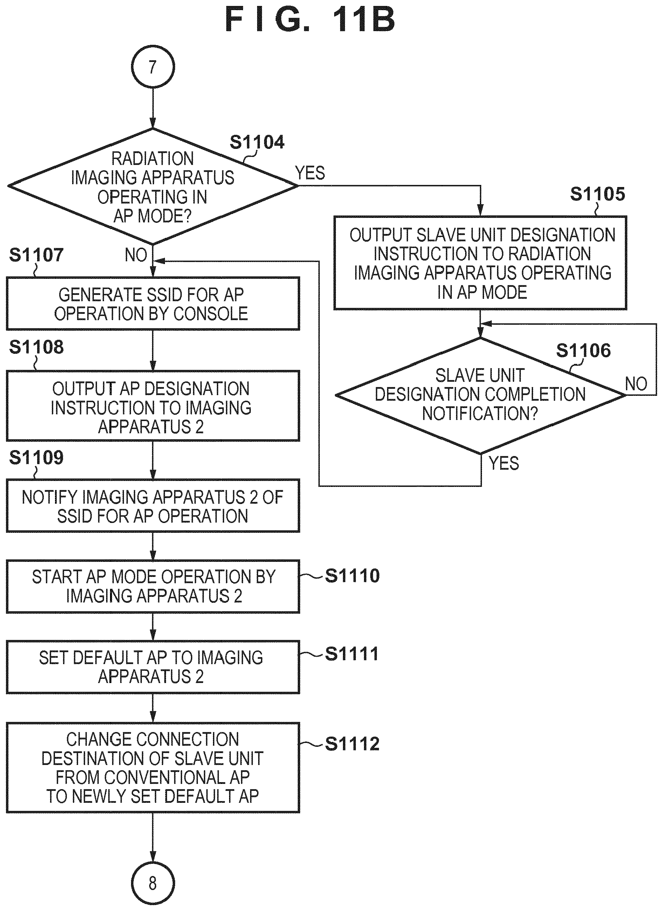

Processing of a radiation imaging system in which a plurality of radiation imaging apparatuses are paired with a console 103 under a condition that only one radiation imaging apparatus operates in the AP mode will be described with reference to Figs. 11A and 11B.

Figs. 11A and 11B are flowcharts based on Fig. 5. The contents of processing after the start of communication preparation until the first radiation imaging apparatus (to be referred to as an imaging apparatus 1 hereinafter) is paired, and the communication preparation is completed are the same as in Fig. 3 (step S1101). In a case in which each unit is activated, and then, pairing of the radiation imaging apparatus (imaging apparatus 1) is performed, if an AP unit 104 does not exist in the radiation imaging system, the console 103 instructs the radiation imaging apparatus (imaging apparatus 1) to preferentially operate in the AP mode (AP designation instruction). On the other hand, if the AP unit 104 exists in the radiation imaging system, the console 103 can control based on a user selection whether to make the radiation imaging apparatus (imaging apparatus 1) to operate as a slave unit or an access point (AP).

In step S1102, pairing of the second radiation imaging apparatus (to be referred to as an imaging apparatus 2 hereinafter) starts. In step S1103, the console 103 determines whether to use the imaging apparatus 2 as a slave unit. The determination processing of step S1103 can be executed by the same processing as the processing (step S309 in Fig. 3) of determining whether to use the imaging apparatus 1 as a slave unit.

If the radiographer as the user selects in step S1103 not to use the imaging apparatus 2 as a slave unit, that is, to make the imaging apparatus 2 operate as an access point (AP: master unit) (NO in step S1103), the process advances to step S1104.

In step S1104, the console 103 determines whether a radiation imaging apparatus operating in the AP mode exists in the radiation imaging system. As the result of the process of step S1101 described above, one of the AP unit 104 and the radiation imaging apparatus (imaging apparatus 1) is set as the default connection destination AP. For this reason, if the radiation imaging apparatus (imaging apparatus 1) is set as the default access point (AP), the console 103 determines in this step that there is a radiation imaging apparatus operating in the AP mode. On the other hand, if the AP unit 104 is set as the default access point (AP), the console 103 determines in this step that there is no radiation imaging apparatus operating in the AP mode.

If a radiation imaging apparatus operating in the AP mode exists (YES in step S1104), the process advances to step S1105. When the process advances to step S1105, the console 103 notifies the radiation imaging apparatus (in this case, the imaging apparatus 1) operating in the AP mode of an instruction (slave unit designation instruction) to make it operate as a slave unit.

In step S1106, the console 103 stands by in a state to wait for reception of a slave unit designation completion notification transmitted from the radiation imaging apparatus (imaging apparatus 1) instructed to be designated as a slave unit (NO in step S1106). If the console 103 receives the slave unit designation completion notification (YES in step S1106), the process advances to step S1107.

On the other hand, if a radiation imaging apparatus operating in the AP mode does not exist (NO in step S1104), the process advances to step S1107.

In step S1107, the console 103 generates an SSID for the AP operation as wireless identification information necessary for the imaging apparatus 2 to operate in the AP mode. The contents of the SSID generated here will be described later.

In step S1108, the console 103 instructs the imaging apparatus 2 to operate in the AP mode (AP designation instruction).

In step S1109, the console 103 notifies the imaging apparatus 2 of the SSID generated in step S1107. In step S1110, the imaging apparatus 2 that has received the instruction and the SSID information from the console 103 starts an operation (AP mode operation) as an access point (AP) using the received SSID. After that, in step S1111, the console 103 sets the default access point (AP) to the imaging apparatus 2.

In step S1112, the console 103 changes the connection destination of the slave unit from the conventional access point to the newly set default access point (default AP). After the process of step S1112, the process advances to step S1116 to complete the communication preparation.

On the other hand, if the radiographer as the user selects in step S1103 to use the imaging apparatus 2 as a slave unit (YES in step S1103), the process advances to step S1113.

In step S1113, the console 103 notifies the imaging apparatus 2 of an instruction (slave unit designation instruction) to make it operate as a slave unit. In step S1114, the console 103 notifies the imaging apparatus 2 of the SSID of the default access point (default AP) (for example, the AP unit 104 or the imaging apparatus 1) as an access point (AP) to be connected. In step S1115, wireless connection is established between the imaging apparatus 2 and the default access point (default AP) (the AP unit 104 or the imaging apparatus 1), and communication preparation is completed (step S1116).

Note that in the processes of steps S1105 and S1106 in Fig. 11B, when the radiation imaging apparatus operating in the AP mode stops the AP mode operation, the console 103 receives a notification (slave unit designation completion notification) representing that the radiation imaging apparatus (imaging apparatus 1) stops the AP mode operation. After receiving the slave unit designation completion notification, the console 103 instructs another radiation imaging apparatus (imaging apparatus 2) to operate in the AP mode (AP designation instruction). That is, the console 103 receives the slave unit designation completion notification from the radiation imaging apparatus operating in the AP mode, and after that, outputs an AP designation instruction to another radiation imaging apparatus to be made to operate as an access point. The console controls the timing of transmitting an instruction to each radiation imaging apparatus in this way, thereby imposing a restriction to make only one radiation imaging apparatus operate in the AP mode without generating a plurality of radiation imaging apparatuses that have the same SSID and operate in the AP mode at the same timing.

Since only one access point (AP) exists in the radiation imaging system, the console 103 need only generate one type of SSID in step S1107. Even if the radiation imaging apparatus operating in the AP mode changes, a common SSID is set for the radiation imaging apparatuses. For this reason, the console 103 need not perform processing of notifying the slave unit of the SSID of the new master unit.

Additionally, in step S1112, the slave unit can easily change the connection destination. For example, as described in the first embodiment, when changing the access point from the AP unit 104 to the radiation imaging apparatus operating in the AP mode, a character string capable of specifying the radiation imaging apparatus is included in the SSID of the radiation imaging apparatus. Each device is set in advance to be preferentially connected to the radiation imaging apparatus if such an SSID is found.

In the processes of steps S1105 to S1112, when switching the access point from the radiation imaging apparatus (imaging apparatus 1) to the radiation imaging apparatus (imaging apparatus 2), the AP mode operation of the radiation imaging apparatus (imaging apparatus 1) stops (step S1106). Then, the radiation imaging apparatus (imaging apparatus 2) of the same SSID starts the AP mode operation (step S1110). Hence, the slave unit recognizes that the access point (AP) has left the radiation imaging system to be absent temporarily and then returned, and starts connection to the radiation imaging apparatus (imaging apparatus 2) operating as a new access point (AP) (step S1112).

On the other hand, if there is a possibility that a plurality of radiation imaging systems exist in the same environment, more specifically, if a plurality of pairs of consoles and radiation imaging apparatuses exist in one hospital, the console 103 can generate a unique SSID for each system in the hospital using at least the identification information (ID) of a console or the identification information (ID) of a radiation imaging apparatus.

Furthermore, upon detecting that a device that has the same SSID as the SSID of the radiation imaging apparatus operating as an access point (AP) and operates as an access point (AP) exists in the same environment, the console 103 can perform processing of making, on the display unit such as a monitor, a notification representing that a plurality of radiation imaging apparatuses operating in the AP mode have the same SSID and stopping the AP mode operation of a target radiation imaging apparatus.

Detection of a plurality of access points (APs) operating by the same SSID can be done using unique information (second wireless identification information) such as a MAC address originally provided for a wireless LAN device. For example, consider a case in which a plurality of devices (radiation imaging apparatuses and some kinds of communication devices in the radiation imaging system) that have the same SSID and are operating as access points (APs) exist in the environment of the radiation imaging system, and the console 103 sets a radiation imaging apparatus to the default connection destination. When the console 103 is going to connect with a radiation imaging apparatus, the connection destination may be selected at random because there are two devices having the same SSID. Alternatively, the connection destination may be switched due to some reason. Hence, the console 103, for example, acquires and holds the MAC address (second wireless identification information) of an access point (AP) connected based on an SSID as first wireless identification information. The console 103 confirms whether a MAC address acquired by the previous connection is the same as the MAC address acquired by the current connection. If only one access point (AP) exists in the environment of the radiation imaging system, the MAC address does not change. However, if a plurality of access points (APs) of the same SSID exist, and the connection switches to a different access point (AP) out of the plurality of access points (APs), the MAC address (second wireless identification information) changes.

As another method, the console 103 can acquire the range of MAC addresses of connectable devices or their MAC addresses themselves, and when a device is newly connected, confirm whether it is a device within the range of MAC addresses or a device corresponding to a MAC address acquired in advance. As for a method of acquiring the MAC address in advance, the console 103 can set the MAC address based on a user input. Alternatively, the MAC address may be set in the storage unit of the console 103 at the time of manufacture, or the console 103 may acquire MAC address information from an AP unit or radiation imaging apparatus at the time of pairing. The methods exemplified here can also be used in combination.

Upon detecting a state in which devices have the same SSID but different MAC addresses, the console makes a notification representing it on the display unit such as a monitor and outputs a notification to stop using, as the default connection destination, the radiation imaging apparatus using the target SSID. As for the processing at this time, the processing according to the above-described embodiments can be applied to switch the radiation imaging apparatus operating as an access point (AP). It is also possible to instruct radiation imaging apparatus of the same SSID to stop the AP mode operation.

In this embodiment, the radiation imaging system has an arrangement including only one radiation imaging apparatus 101 operating as an access point (AP). However, when activating the console, the user can select whether to employ the operation of this embodiment or an operation as in the first to third embodiments.

According to this embodiment, it is possible to prevent a plurality of access points having the same SSID from being generated in the system.

### (Fifth Embodiment)

In each of the above-described embodiments, an arrangement that generates a unique SSID when pairing a radiation imaging apparatus and a console has been described. However, an SSID for the AP operation may be assigned to a radiation imaging apparatus at the time of manufacture/shipment from the factory, and if a condition is met at the time of pairing, the radiation imaging apparatus may be caused to start an operation as an access point using the SSID for the AP operation. If the number of digits of SSIDs is smaller as compared to the number of manufactured radiation imaging apparatuses, the radiation imaging apparatuses may be divided according to a condition such as the shipment country or region or the user, and an SSID that is unique in each area may be assigned.

According to the above-described embodiments, it is possible to provide a radiation imaging technique capable of performing stable communication between a radiation imaging apparatus and a control apparatus.

### Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

A radiation imaging system includes a radiation imaging apparatus capable of operating as a master unit or a slave unit in wireless communication, and a control apparatus capable of controlling an operation of the radiation imaging apparatus. The control apparatus includes a determination unit configured to determine whether a device operating as the master unit of the wireless communication exists in the radiation imaging system, and a control unit configured to control the operation of the radiation imaging apparatus based on the determination. The control unit controls the operation of the radiation imaging apparatus based on the determination such that one master unit of the wireless communication exists in the radiation imaging system.

This application is a divisional application of European patent application no. 16 178 945.8 (the "parent application"), also published as EP 3 132 746. Based on the original claims of the parent application, the following aspects form part of the content of this divisional application as filed.
1. A radiation imaging system including a radiation imaging apparatus capable of operating as one of master means and slave means in wireless communication, and a control apparatus capable of controlling an operation of the radiation imaging apparatus, the control apparatus comprising:
   determination means for determining whether a device operating as the master means of the wireless communication exists in the radiation imaging system; and
   control means for controlling the operation of the radiation imaging apparatus based on the determination,
   wherein the control means controls the operation of the radiation imaging apparatus based on the determination such that one master means of the wireless communication exists in the radiation imaging system.
2. The system according to aspect 1, further comprising generation means for generating unique wireless identification information,
   wherein the control means controls the operation of the radiation imaging apparatus as the master means of the wireless communication based on the unique wireless identification information.
3. The system according to aspect 2, wherein the generation means generates the unique wireless identification information based on character information capable of identifying the device operating as the master means of the wireless communication, unique identification information of the control apparatus, and unique identification information of the radiation imaging apparatus.
4. The system according to aspect 1, wherein if the device operating as the master means does not exist based on the determination, the control means makes the radiation imaging apparatus operate as the master means of the wireless communication, and
   if the device operating as the master means exists, the control means makes the radiation imaging apparatus operate as the slave means of the wireless communication based on wireless identification information of the device.
5. The system according to aspect 1, wherein the control apparatus further comprises selection means for selecting, based on an operation input, whether to make the radiation imaging apparatus operate as the slave means or the master means of the wireless communication,
   wherein the control means controls the operation of the radiation imaging apparatus based on the selection.
6. The system according to aspect 5, wherein if the selection means selects to make the radiation imaging apparatus operate as the slave means of the wireless communication, the control means notifies the radiation imaging apparatus of wireless identification information of the device operating as the master means and makes the radiation imaging apparatus operate as the slave means of the device.
7. The system according to aspect 5, wherein if the selection means selects to make the radiation imaging apparatus operate as the master means, the control means notifies the radiation imaging apparatus of unique wireless identification information and makes the radiation imaging apparatus operate as the master means of the wireless communication.
8. The system according to aspect 7, wherein the control means changes a default connection destination of the master means in the radiation imaging system to the radiation imaging apparatus.
9. The system according to aspect 1, wherein if a wireless communication intensity between the radiation imaging apparatus and the control apparatus becomes lower than a threshold, the control means makes the radiation imaging apparatus operating as the master means operate as the slave means.
10. The system according to aspect 9, wherein if the wireless communication is not performed between the radiation imaging apparatus and the control apparatus for not less than a predetermined time, the control means makes the radiation imaging apparatus operating as the master means operate as the slave means.
11. The system according to aspect 5, wherein if a radiation imaging apparatus is newly added to the radiation imaging system in which the radiation imaging apparatus operates as the master means of the wireless communication,
   the selection means selects whether to make the added radiation imaging apparatus operate as the slave means or the master means of the wireless communication, and
   the control means controls the operation of the added radiation imaging apparatus based on the selection.
12. The system according to aspect 11, wherein if the selection means selects to make the added radiation imaging apparatus operate as the master means,
   the control means makes the radiation imaging apparatus operating as the master means operate as the slave means of the wireless communication, and then makes the added radiation imaging apparatus operate as the master means of the wireless communication based on unique wireless identification information.
13. The system according to aspect 11, wherein if the selection means selects to make the added radiation imaging apparatus operate as the slave means,
   the control means notifies the added radiation imaging apparatus of wireless identification information of the radiation imaging apparatus operating as the master means, and makes the added radiation imaging apparatus operate as the slave means of the wireless communication.
14. A method of controlling a radiation imaging system including a radiation imaging apparatus capable of operating as one of master means and slave means in wireless communication, and a control apparatus capable of controlling an operation of the radiation imaging apparatus, comprising:
   determining whether a device operating as the master means of the wireless communication exists in the radiation imaging system; and
   controlling the operation of the radiation imaging apparatus based on the determination,
   wherein in the controlling, the operation of the radiation imaging apparatus is controlled based on the determination such that one master means of the wireless communication exists in the radiation imaging system.
15. A control apparatus for controlling an operation of a radiation imaging apparatus capable of operating as one of master means and slave means in wireless communication, comprising:
   determination means for determining whether a device operating as the master means of the wireless communication exists; and
   control means for controlling the operation of the radiation imaging apparatus based on the determination,
   wherein the control means controls the operation of the radiation imaging apparatus based on the determination such that one master means of the wireless communication exists in a radiation imaging system including the radiation imaging apparatus.

## Claims

1. A radiation imaging system (100) including a radiation imaging apparatus (101) which is an apparatus that performs radiation imaging by detecting intensity distribution of radiation transmitted through a subject and converting the intensity distribution into image data, the apparatus being capable of operating as an access point in wireless communication which has unique wireless identification information, and a control apparatus (103) capable of controlling an operation of the radiation imaging apparatus and receiving the image data via the wireless communication, the control apparatus comprising:
generation means for generating the unique wireless identification information, wherein the unique wireless identification information includes character information indicating that the radiation imaging apparatus operates as the access point, unique identification information of the radiation imaging apparatus, and unique identification information of the control apparatus; and
control means for controlling the operation of the radiation imaging apparatus based on the unique wireless identification information.

2. The system according to claim 1, wherein the unique wireless identification information further includes information for specifying a timing of pairing between the radiation imaging apparatus and the control apparatus.

3. The system according to claim 2, further comprising determination means for determining whether a device operating as the access point exists in the radiation imaging system.

4. The system according to claim 3, wherein if the device operating as the access point does not exist based on the determination, the control means is configured to make the radiation imaging apparatus operate as the access point , and
if the device operating as the access point exists, the control means is configured to not make the radiation imaging apparatus operate as the access point based on wireless identification information of the device.

5. The system according to claim 1, wherein the control apparatus further comprises selection means for selecting, based on an operation input, whether to make the radiation imaging apparatus operate as the access point,
wherein the control means is configured to control the operation of the radiation imaging apparatus based on the selection.

6. The system according to claim 5, wherein if the selection means selects not to make the radiation imaging apparatus operate as the access point, the control means is configured to notify the radiation imaging apparatus of wireless identification information of the device operating as the access point and make the radiation imaging apparatus operate using the device as the access point.

7. The system according to claim 5, wherein if the selection means selects to make the radiation imaging apparatus operate as the access point, the control means is configured to notify the radiation imaging apparatus of the unique wireless identification information and make the radiation imaging apparatus operate as the access point.

8. The system according to claim 7, wherein the control means is configured to change a default connection destination of the access point in the radiation imaging system to the radiation imaging apparatus.

9. The system according to claim 2, wherein if a wireless communication intensity between the radiation imaging apparatus and the control apparatus becomes lower than a threshold, the control means is configured to not make the radiation imaging apparatus operate as the access point.

10. The system according to claim 9, wherein if the wireless communication is not performed between the radiation imaging apparatus and the control apparatus for not less than a predetermined time, the control means is configured to not make the radiation imaging apparatus operate as the access point.

11. The system according to claim 5, wherein if a radiation imaging apparatus is newly added to the radiation imaging system in which the radiation imaging apparatus operates as the access point,
the selection means is configured to select whether to make the added radiation imaging apparatus operate as the access point, and
the control means is configured to control the operation of the added radiation imaging apparatus based on the selection.

12. The system according to claim 11, wherein if the selection means selects to make the added radiation imaging apparatus operate as the access point,
the control means is configured to not make the radiation imaging apparatus operating as the access point operate as the access point, and then makes the added radiation imaging apparatus operate as the access point based on the unique wireless identification information.

13. The system according to claim 11, wherein if the selection means selects not to make the added radiation imaging apparatus operate as the access point,
the control means is configured to notify the added radiation imaging apparatus of the unique wireless identification information of the radiation imaging apparatus operating as the access point, and not make the added radiation imaging apparatus operate as the access point.

14. A method of controlling a radiation imaging system including a radiation imaging apparatus which is an apparatus that performs radiation imaging by detecting intensity distribution of radiation transmitted through a subject and converting the intensity distribution into image data, and which is an apparatus capable of operating as an access point in wireless communication which has unique wireless identification information, and a control apparatus capable of controlling an operation of the radiation imaging apparatus and receiving the image data via the wireless communication, the method comprising:
generating the unique wireless identification information including unique identification information of the control apparatus, wherein the unique wireless identification information includes character information indicating that the radiation imaging apparatus operates as the access point, unique identification information of the radiation imaging apparatus, and unique identification information of the control apparatus; and
controlling the operation of the radiation imaging apparatus based on the unique wireless identification information.

15. A control apparatus for controlling an operation of a radiation imaging apparatus and receiving image data via wireless communication from the radiation imaging apparatus which is an apparatus that performs radiation imaging by detecting intensity distribution of radiation transmitted through a subject and converting the intensity distribution into the image data, and which is an apparatus capable of operating as an access point in wireless communication that has unique wireless identification, comprising:
generation means for generating the unique wireless identification information, wherein the unique wireless identification information includes character information indicating that the radiation imaging apparatus operates as the access point, unique identification information of the radiation imaging apparatus, and unique identification information of the control apparatus; and
control means for controlling the operation of the radiation imaging apparatus based on the unique wireless identification information.
